# EUROPEAN PATENT APPLICATION

(11) **EP 4 086 341 A1**
(43) Date of publication of application: **09.11.2022**
(21) Application number: 20911101.2
(22) Date of filing: 30.12.2020
(51) Int. Cl.: C12N 5/10, C12N 15/113, C07K 16/28, A61K 35/17

(54) **METHOD FOR PURIFYING UCART CELL AND USE THEREOF**

(30) Priority: 30.12.2019 WO PCT/CN2019/129955
(71) Applicant: Edigene Biotechnology Inc., Beijing 102206 (CN)
(72) Inventor: YUAN, Pengfei, Beijing 102206 (CN); WANG, Fei, Beijing 102206 (CN); NIU, Lichao, Beijing 102206 (CN); FU, Jianqiang, Beijing 102206 (CN); GU, Feng, Beijing 102206 (CN)
(74) Representative: Lavoix
(86) International application number: PCT/CN2020/141566
(87) International publication number: WO 2021/136415

(57) **Abstract**

A method for purifying a universal human CAR-T cell, comprising: (i) destroying, by using gene editing technology, a TRAC gene region from position 23016448 to position 23016490 of chromosome 14 and a B2M gene region from position 45003745 to position 4500378 of chromosome 15 in the human CAR-T cell; (ii) then introducing a mRNA targeting TCRα/β into the gene-edited CAR-T cell; and (iii) *in vitro* culturing the CAR-T cell population after the described treatment.

## Description

### FIELD OF THE INVENTION

This application relates to a transiently expressed CAR that targets T cells, and specifically relates to addition of allogeneic CAR-T cells targeting tumor cells after *in vitro* culture in combination with a double-gene knockout technology of gene editing, and during the culture process, a transiently expressed mRNA targeting TCRα/β is added, so as to obtain a method for preparing universal CAR-T cells, over about 97% of which are TCRα/β negative.

### BACKGROUND OF THE INVENTION

Malignant tumors have become a serious threat to health and life safety, and the cure of tumors has always been the dream of mankind. In recent years, tumor immunotherapy has received extensive attention, especially the emergence of CAR-T (Chimeric Antigen Receptor T Cells) technology has made a milestone development in tumor control. It is from 1989, the first application of CAR-T technology , to 2012, when Emily Whitehead, who was cured by a team led by Professor Carl June of the University of Pennsylvania, and to 2017 when the FDA Oncology Drug Advisory Committee (ODAC) unanimously recommended approval of Novartis CAR-T drug CTL019 for the treatment of juvenile advanced B-cell acute lymphoblastic leukemia (r/rAll) by an overwhelming vote of 10:0.

Generally, T cells in traditional CAR-T technology are mainly derived from patients themselves. In a GMP environment, T cells are isolated *in vitro,* activated, CAR introduced, cultured and expanded, and finally reinfused to the patient after quality control. Due to the influence of the patient's own conditions, problems such as being unsuitable for blood collection or difficult to expand the T cells after blood collection and their isolation will follow. When the patient is in critical condition, from the isolation of T cells to the reinfusion of CAR-T, the waiting time of the whole process will also be a major problem for autologous reinfusion. These problems have brought limitations to the wide application of CAR-T technology. Therefore, an important research direction of CAR-T cell therapy is how to use the T cells of a healthy blood donor to prepare a large number of CAR-T cells to meet the clinical needs of patients. The establishment of this technology will greatly reduce the cost of CAR-T therapy, better ensure the quality of uniformly prepared cells, and patients can get CAR-T cells for treatment immediately when needed. At the same time, allogeneic CART therapy also has its disadvantages, such as easily causing GvHD, and the means by using antibody purification technology in the prior art is costly, complex, time-consuming and labor-intensive. Therefore, the establishment of new purification methods is particularly urgent.

Several documents are cited throughout the text of this specification. Each document herein (including any journal articles or abstracts, published or unpublished patent applications, issued patents, manufacturer's instructions, instructions for use, etc.) is hereby incorporated by reference. However, there is no admission that the documents cited herein are in fact prior art to the present invention.

### SUMMARY OF THE INVENTION

The technical solutions of the present application are implemented to solve the above problems existing in the art. This application uses the CRISPR/Cas9 system to conduct double-gene (TRAC and B2M) knock out in CAR-T cells, and the knockout efficiency is as high as 80%. However, nearly 20% of positive CAR-T cells still express TCRα/β that can cause GvHD. Therefore, using the electroporation delivery platform to transiently transfer CAR targeting TCR α/β into CAR-T cells can not only save the purification step, but also save manpower, material and financial resources. At the same time, for the targets for targeting TCRα/β of T cells, this application is the first publication which provides a method for the establishment of gene editing technology combined with adoptive immunization in the treatment of tumors and viral infectious diseases (such as HIV/AIDS), and has laid a solid technical foundation for the research on the treatment of related diseases.

Therefore, the present application provides efficient knockout in CAR-T cells using gene editing technology, such as CRISPR/Cas9 system, so as to obtain gene-edited CAR-T cells, and at the same time, the expressed CAR targeting TCRα/β is transiently transferred into CAR-T cells using the electroporation system (except TCRα/β CAR). At this time, the transiently transfected CAR can clear the TCRα/β-positive group of cells, thereby directly obtaining universal CAR-T cells. This method provides a cost-effective, labor-saving and time-saving method in addition to the traditional method for removing TCRα/β-positive cell populations.

Specifically, this application provides the following:
1. A method for purifying universal human CAR-T cells, comprising:
   (i) disrupting a TRAC genomic region and a B2M genomic region in a human CAR-T cell by gene editing technology; and
   (ii) introducing an mRNA targeting TCRα/β into the gene-edited CAR-T cell of step (i).
2. The method according to claim 1, wherein the mRNA targeting TCRα/β is introduced into the CAR-T cell by transient transfection.
3. The method according to claim 1 or 2, further comprising culturing a cell population obtained by the method *in vitro* for an appropriate time to eliminate TCRα/β positive cells.
4. The method according to claim 3, wherein the appropriate time is from 4 to 8 days, preferably from 5 to 7 days, most preferably 6 days.
5. The method according to any one of claims 1 to 4, wherein the TRAC genomic region comprises the genomic region of human chromosome 14 from position 23016448 to 23016490, and the B2M genomic region comprises the genomic region of human chromosome 15 from position 45003745 to 45003788.
6. The method according to any one of claims 1 to 5, wherein the mRNA targeting TCRα/β encodes an anti-TCRα/β scFv molecule, wherein,
   a light chain variable region of the anti-TCRα/β scFv molecule is shown in SEQ ID NO: 19 
(QIVLTQSPAIMSASPGEKVTMTCSATSSVSYMHWYQQKSGTSPKRWIYDTSKLASGV
PARFSGSGSGTSYSLTISSMEAEDAATYYCQQWSSNPLTFGAGTKLELK);
 and
   a heavy chain variable region of the anti-TCRα/β scFv molecule is shown in SEQ ID NO: 20 
(EVQLQQSGPELVKPGASVKMSCKASGYKFTSYVMHWVKQKPGQGLEWIGYINPYN
DVTKYNEKFKGKATLTSDKSSSTAYMELSSLTSEDSAVHYCARGSYYDYDGFVYWG
QGTLVTVSA).
7. The method according to claim 6, wherein the light chain variable region of the scFv molecule and the heavy chain variable region of the scFv molecule are connected by a linker, and the linker is an amino acid sequence comprising (G)n(S)m, wherein, n is a positive integer from 1 to 20, and m is a positive integer from 1 to 10.
8. The method according to claim 7, wherein the sequence of the linker is shown in SEQ ID NO: 21: GGGGSGGGGSGGGGSGGGGS.
9. The method according to any one of claims 1 to 8, wherein the amino acid sequence encoded by the mRNA targeting TCRα/β is shown in SEQ ID NO: 14: 
QIVLTQSPAIMSASPGEKVTMTCSATSSVSYMHWYQQKSGTSPKRWIYDTSKLAS
GVPARFSGSGSGTSYSLTISSMEAEDAATYYCQQWSSNPLTFGAGTKLELKGGGGSGG
GGSGGGGSGGGGSEVQLQQSGPELVKPGASVKMSCKASGYKFTSYVMHWVKQKPG
QGLEWIGYINPYNDVTKYNEKFKGKATLTSDKSSSTAYMELSSLTSEDSAVHYCARGS
YYDYDGFVYWGQGTLVTVSA.
10. The method according to any one of claims 1 to 9, wherein the TRAC genomic region and the B2M genomic region are disrupted by homologous recombination, zinc finger nucleases gene editing technology, TALEN gene editing technology or CRISPR/Cas gene editing technology.
11. The method according to claim 10, wherein the gene editing technology is CRISPR/Cas9 gene editing technology.
12. The method according to claim 11, comprising:
   (i) introducing a guide RNA (sgRNA) targeting the TRAC genomic region into a T cell to edit the TRAC genomic region; and/or
   (ii) introducing a guide RNA (sgRNA) targeting the B2M genomic region into a T cell to edit the B2M genomic region.
13. The method according to claim 12, wherein:
   (i) the guide RNA (sgRNA) targeting the TRAC genomic region comprises a sequence selected from the group consisting of: SEQ ID NOs: 2 to 5; and
   (ii) the guide RNA (sgRNA) targeting the B2M genomic region comprises a sequence selected from the group consisting of: SEQ ID NOs: 6 to 13.
14. The method according to claim 13, comprising:
   (i) introducing the sgRNA encoding the TRAC genomic region and the Cas9-encoding nucleotides concurrently or separately into a T cell to edit the TRAC genomic region; and/or
   (ii) introducing the sgRNA encoding the B2M genomic region and the Cas9-encoding nucleotides concurrently or separately into a T cell to edit the B2M genomic region.
15. The method according to any one of claims 12-14, wherein the sgRNA is chemically modified.
16. The method according to claim 15, wherein the chemical modification comprises a 2'-O-methyl modification or an internucleotide 3' thiol modification.
17. The method according to claim 16, wherein the chemical modification is a 2'-O-methyl modification at the first one, two and/or three bases at the 5' end and/or the last base at the 3' end of the sgRNA.
18. The method according to any one of claims 14 to 17, wherein the sgRNA and the Cas9-encoding nucleotide sequence are introduced into the T cell together by electroporation.
19. The method according to claim 18, wherein a condition of the electroporation is selected from any one of the following conditions: 150-250V, 0.5-2ms; 150V, 2ms; 160V, 2ms; 170V, 2ms; 180V, 2ms; 190V, 1ms; 200V, 1ms; 210V, 1ms; 220V, 1ms; 230V, 1ms; 240V, 1ms; or 250V, 0.5ms.
20. The method according to any one of claims 1 to 19, wherein the TRAC and B2M are independently knocked out with an efficiency of more than 90%; and collectively knocked out with an efficiency of more than 75%.
21. The method according to any one of claims 1 to 20, wherein, the ratio of TCR-negative cells accounts for 98% or more than 99% of all CAR-T cells in the cell population obtained by the method.
22. A purified universal human CAR-T cell, wherein:
   (i) the TRAC genomic region and the B2M genomic region in the CAR-T cell have been disrupted by gene editing;
   (ii) an mRNA targeting TCRα/β has been introduced into the CAR-T cell.
23. The universal human CAR-T cell according to claim 22, which expresses at a low level or does not express one or more proteins selected from the group consisting of: TCRα/β, HLA class 1 protein, PD-1, TIM3, and LAG3.
24. A biological product comprising the universal human CAR-T cell of any one of claims 22 or 23.
25. The biological product according to claim 24, wherein 98% or more than 99% of the universal human CAR-T cells are TCR negative.
26. Use of the universal human CAR-T cell according to any one of claims 22 or 23 or the biological product according to any one of claims 24 or 25 in the preparation of a medicament for treating a disease in a subject.
27. A method for treating a disease in a subject, comprising administering to the subject an effective amount of the universal human CAR-T cell according to claim 22 or 23 or the biological product according to claim 24 or 25.
28. The method according to claim 27, wherein the disease is tumor.
29. The method according to claim 28, wherein the tumor is hematological tumor.
30. The method according to claim 29, wherein the tumor is lymphoma or a leukemia.

### BRIEF DESCRIPTION OF THE DRAWINGS

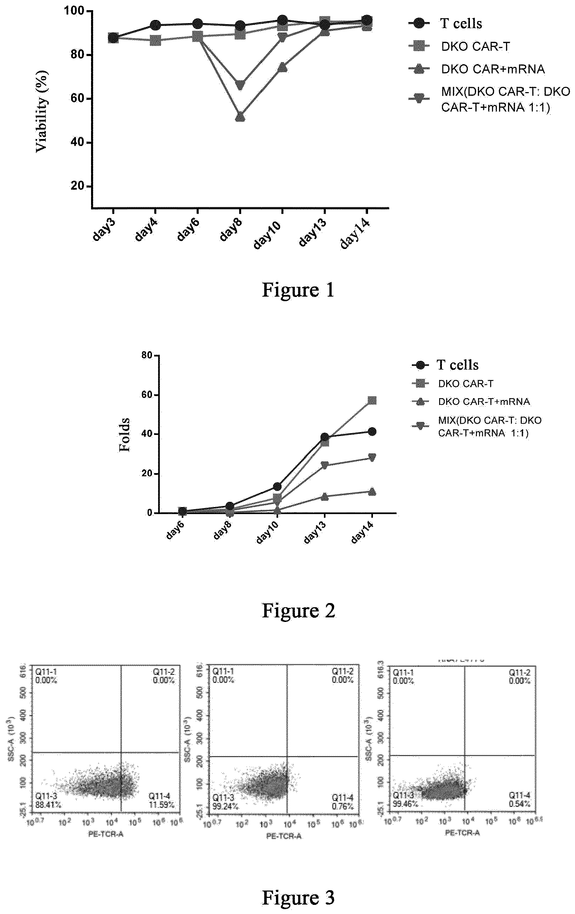

Figure 1 shows the changes in the growth and viability of cells after electroporation. The viability of T cells and DKO CAR-T cells is relatively good, and the cell viability reaches more than 90% with the increase of culture time, while after the second electroporation of DKO CAR-T+mRNA cells, its viability is significantly reduced, and its viability gradually recovers with the increase of culture time. The viability of MIX (DKO CAR-T: DKO CAR-T+mRNA 1:1) cells also decrease significantly after the sixth day of mixed culture, and its viability gradually recovers with the increase of culture time. After 14 days of culture, the viability basically reaches more than 90%.
Figure 2 shows the growth of each group after the cells are electroporated. The proliferation rate of T cells in the early stage is the fastest, after 13 days the proliferation rate slows down. The growth trend of DKO CAR-T cells is basically the same as that of T cells, till the 14th day of culture, its proliferation rate keeps basically the same. DKO CAR-T+ mRNA cells are the edited T cells which are electroporated for a second time when cultured to the sixth day, and its growth rate is slow. MIX is a 1:1 mixed culture of DKO CAR-T cells and DKO CAR-T+mRNA cells electroporated for the second time when cultured to the sixth day, and the proliferation rate after mixed culture is between that of the DKO CAR-T cells and that of the DKO CAR-T+mRNA cells.
Figure 3 shows the phenotypic analysis of gene-edited CAR-T cells cultured after the introduction of TCRα/β-targeted-CAR mRNA. It can be seen from the results that after the DKO-T cells added with TCRα/β-targeted-CAR mRNA are cultured, the proportion of TCRα/β negative cells is no less than 99%.

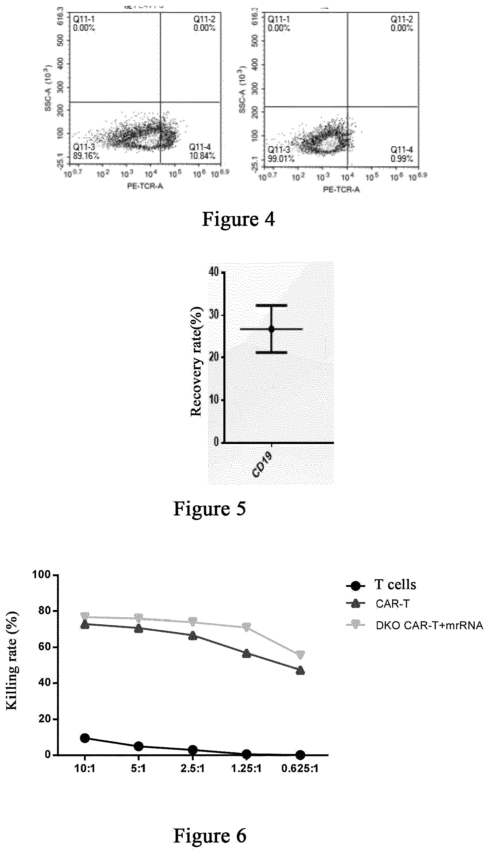

Figure 4 shows the changes of TCRα/β positive cells after MIX (DKO CAR-T:DKO CAR-T+mRNA 1:1) cells are cultured. After 2 days of mixed culture, the proportion of TCRα/β negative cells is no less than 99%.
Figure 5 shows the recovery rate of purified TRAC/B2M knockout T cells (DKO CAR-T) using STEMCELL to screen and purify. It can be seen from the figure that the recovery rate is low, and the recovery rate is basically between 20% and 30%.
Figure 6 shows the validation and result comparison of the cell killing function of T cells, CAR-T cells and DKO CAR-T+mRNA cells. In this experiment, T cells, CAR-T cells and DKO CAR-T+mRNA cells are used as effector cells, and the *in vitro* killing experiments are carried out with effector-target ratios of 10:1, 5:1, 2.5:1, 1.25:1, and 0.625:1.

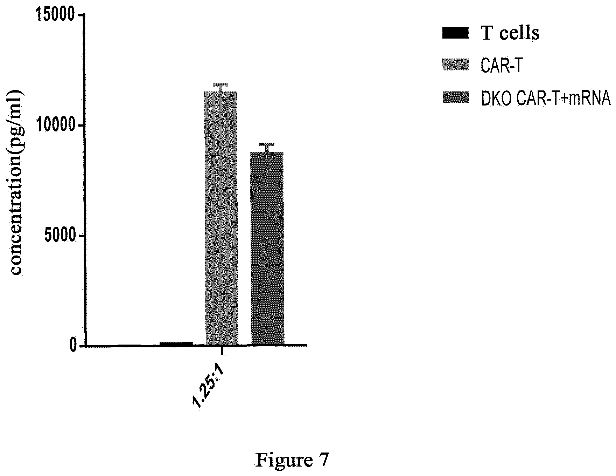

Figure 7 shows the release of IFN-γ factor from T cells, CAR-T cells and DKO CAR-T+mRNA cells. In this experiment, Raji is used as the target cell, and T cells, CAR-T cells and DKO CAR-T+mRNA cells are used as effector cells. After co-culturing *in vitro* with the effector-target ratios of 10:1, 5:1, 2.5:1, 1.25:1, and 0.625:1, 1.25:1 supernatant is taken to detect IFN-γ.

### DETAILED DESCRIPTION OF THE INVENTION

The present application will be further described in detail below in conjunction with the specific embodiments, and the examples are given to enable a more thorough understanding of the present application and to fully convey the scope of the present application to those skilled in the art.

It should be noted that certain terms are used in the specification and claims to refer to specific components. It should be understood by those skilled in the art that the same component may be referred to by different nouns. The specification and the claims do not use the difference in terms as a way to distinguish components, but use the difference in function of the components as a criterion for distinguishing. As referred to throughout the specification and claims, "comprising" or "including" is an open-ended term and should be interpreted as "including but not limited to". Subsequent descriptions in the specification are preferred embodiments for implementing the present application, however, the descriptions are still for the purpose of general principles of the specification and are not intended to limit the scope of the present application. The scope of protection of the present application should be determined by the appended claims. A first aspect of the present application provides a method for purifying universal human CAR-T cells, in a specific embodiment, the method comprises:
(i) disrupting a TRAC genomic region and a B2M genomic region in a human CAR-T cell by gene editing technology; and
(ii) introducing an mRNA targeting TCRα/β into the gene-edited CAR-T cell of step (i).

"CAR-T" is an abbreviation for "Chimeric Antigen Receptor T Cells", in which the Chimeric Antigen Receptor (CAR) is the core part of CAR-T, which enables T cells to recognize target cell (e.g. tumor) antigens in an HLA-independent manner, which allows CAR-engineered T cells to recognize a wider range of targets than the native T-cell surface receptor TCR. In some embodiments, the tumor-targeted CAR is designed to include a tumor-associated antigen (TAA) binding region (e.g., an scFV segment typically derived from the antigen binding region of a monoclonal antibody), an extracellular hinge region, a transmembrane domain and an intracellular signaling domain. The choice of target antigen is a key determinant for the specificity and efficacy of CAR, and safety of the genetically modified T cells themselves.

"Universal CAR-T cells" refer to CAR-T cells that can target specific target cell (such as tumor)-related markers and the TCR and MHC on the cell surface of which are functionally inactivated, reducing the immune rejection caused by allogeneic cell therapy.

CAR-T treatment using autologous cells requires blood extraction to isolate the patient's own T lymphocytes for preparation. On the one hand, due to the different conditions of the patient and the different state of T lymphocytes, there are many factors affecting the production process of CAR-T, and thus the production cannot be standardized which affects the safety. On the other hand, some patient's autologous T lymphocytes are insufficient in activity and number after chemotherapy, or are affected by the tumor environment, resulting in limited T lymphocyte activity and proliferation. Such cells are often difficult to be prepared as CAR-T, and the safety and effectiveness of the treatment is compromised. In emergency for preparation of CAR-T cells, the prepared cells cannot be infused back to the patient in time, which will also affect the therapeutic effect. And possibly being affected by the state of autologous T lymphocytes, some tumor patients cannot receive autologous CAR-T cell adoptive therapy. Universal CAR-T or universal T lymphocytes that can be used for allogeneic therapy have a great advantage in above-mentioned situations.

Adoptive cellular therapy (ACT), or adoptive immunotherapy, such as tumor adoptive immunotherapy, refers to a treatment method that treats immune cells *in vitro,* such as adding specific antigens, modifying molecules expressed by immune cells or stimulating them with cytokines, etc., screens and massively expands highly specific target cell (such as tumor)-killing immune effector cells, and then infused them into patients to kill target cells (such as tumors). It is passive immunotherapy.

In the present application, T cells are derived from healthy humans. In some embodiments, the T cells are derived from a patient, such as a cancer patient, e.g., a cancer patient prior to chemotherapy or radiation therapy. In some embodiments, the T cells are derived from umbilical cord blood, bone marrow, or peripheral blood mononuclear cells (PBMCs). In some embodiments, the T cells are derived from stem cells, such as hematopoietic stem cells at various stages of differentiation. The preparation methods described in this application can be used to knock out TRAC, B2M in, for example, PBMC or stem cells and further culture, differentiate, and/or purify corresponding genetically engineered T cells.

A "T cell receptor (TCR)" in this application is a characteristic marker on the surface of all T cells that binds to CD3 to form a TCR-CD3 complex. TCR is composed of two peptide chains, α and β, and each peptide chain can be divided into variable region (V region), constant region (C region), transmembrane region and cytoplasmic region. The TCR molecule belongs to the immunoglobulin superfamily, and its antigen specificity exists in the V region; the V regions (Vα, Vβ) each has three hypervariable regions, CDR1, CDR2, and CDR3. Among them, CDR3 has the most variation, which directly determines the antigen binding specificity of TCR. When TCR recognizes the MHC-antigen peptide complex, CDR1 and CDR2 recognize and bind to the side wall of the antigen-binding groove of the MHC molecule, while CDR3 directly binds to the antigen peptide. TCRs are divided into two categories: TCR1 and TCR2; TCR1 is composed of two chains, γ and δ, and TCR2 is composed of two chains, α and β. In peripheral blood, 90%-95% of T cells express TCR2; and any one of T cells expresses only one of TCR2 and TCR1.

"β2 microglobulin (B2M)" is the β chain (light chain) part of human leukocyte antigen (HLA) on cell surface, and is a single-chain polypeptide with a molecular mass of 11800 and composed of 99 amino acids.

In a specific embodiment of the present application, the coding nucleotides targeting TCRα/β are mRNA, such as an mRNA comprising an ARCA cap. In some embodiments, the mRNA is delivered into the CAR-T cells using an electroporation delivery system. The transiently expressed protein targeting TCRα/β can specifically recognize TCRα/β-positive cells, and achieve the effect of eliminating TCRα/β-positive cells and avoiding the generation of GvHD in the process of culture.

Transient transfection is one of the ways to introduce mRNA into eukaryotic cells. In transient transfection, recombinant mRNA is introduced into a highly infectious cell line to obtain transient but high-level expression of the gene of interest. The transfected mRNA need not to be integrated into the host chromosome. The mRNA introduced by transient transfection is free in the cell and is not embedded in the genome. The transfected cells can be harvested in a shorter time than that using stable transfection, and higher expression efficiency can be obtained in a shorter time. The transient expression method has many advantages, such as simple operation and short period, high expression efficiency, safety and efficiency, and no need for gene screening.

A "graft-versus-host reaction (GvHD)" refers to a reaction due to the presence of immunogenetic differences between the donor and recipient, e.g., on the one hand, when donor cells, such as immunocompetent T lymphocytes from a donor, enter the recipient patient's body and proliferate to a certain extent, the normal cells or tissues of the recipient patient are mistaken for the target to attack. On the other hand, as allogeneic cells, the recipient body's normal immune system may also clear them to produce a "host-versus-graft response (HVGR)".

HVGR and GVHR-related genes include TCR and HLA molecule-related genes. T lymphocytes knocked out of these genes collectively will not cause graft-versus-host disease (GvHD) when they are reinfused into allogeneic patients, so they can be referred to as "universal T cells". For example, a single TRAC gene is a TCRα chain encoding gene which forms a complete functional TCR complex with the two TRBC genes encoding TCRβ. Knockout of TRAC can cause TCR inactivation, and B2M is a MHC I related gene. T lymphocytes collectively knocked out of both genes do not cause graft-versus-host disease (GvHD) when reinfused into allogeneic patients.

In a specific embodiment, the method further includes culturing the obtained cell population *in vitro* for an appropriate time to eliminate the TCRα/β-positive cell population. In a preferred embodiment, the time of *in vitro* culture is from 4 to 8 days, more preferably from 5 to 7 days, and most preferably 6 days.

In a specific embodiment, the mRNA targeting TCRα/β encodes an anti-TCRα/β scFv molecule. "scFv" refers to single chain antibody fragment, which is formed by connecting the heavy chain variable region and the light chain variable region of the antibody through a short peptide (i.e., linker) of 15 to 20 amino acids. The scFv molecule can be regarded as the extracellular part of the CAR molecule, which is composed of the light chain variable region, the linker and the heavy chain variable region.

In a specific embodiment, the light chain variable region of the anti-TCRα/β scFv molecule is shown in SEQ ID NO: 19 (QIVLTQSPAIMSASPGEKVTMTCSATSSVSYMHWYQQKSGTSPKRWIYDTSKLASGV PARFSGSGSGTSYSLTISSMEAEDAATYYCQQWSSNPLTFGAGTKLELK); the heavy chain variable region of the anti-TCRα/β scFv molecule is shown in SEQ ID NO: 20 (EVQLQQSGPELVKPGASVKMSCKASGYKFTSYVMHWVKQKPGQGLEWIGYINPYN DVTKYNEKFKGKATLTSDKSSSTAYMELSSLTSEDSAVHYCARGSYYDYDGFVYWG QGTLVTVSA).

Further, the light chain variable region of the scFv molecule and the heavy chain variable region of the scFv molecule are connected by a linker, and the linker is an amino acid sequence comprising (G)n(S)m, wherein n is a positive integer selected from 1 to 20, m is a positive integer selected from 1 to 10. Specifically, n can be 1, 2, 3, 4, 5, 6, 7, 8, 9, 10, 11, 12, 13, 14, 15, 16, 17, 18, 19, 20; and m can be 1, 2, 3, 4, 5, 6, 7, 8, 9, 10. Herein, G is the abbreviated form of glycine, which can also be abbreviated as Gly; herein, S is the abbreviated form of serine, which can also be abbreviated as Ser.

Further, the linker sequence is shown in SEQ ID NO: 21 (GGGGSGGGGSGGGGSGGGGS).

In a specific embodiment, the amino acid sequence encoded by the mRNA targeting TCRα/β is shown in SEQ ID NO: 14

(QIVLTQSPAIMSASPGEKVTMTCSATSSVSYMHWYQQKSGTSPKRWIYDTSKLASGV
PARFSGSGSGTSYSLTISSMEAEDAATYYCQQWSSNPLTFGAGTKLELKGGGGSGGGG
SGGGGSGGGGSEVQLQQSGPELVKPGASVKMSCKASGYKFTSYVMHWVKQKPGQG
LEWIGYINPYNDVTKYNEKFKGKATLTSDKSSSTAYMELSSLTSEDSAVHYCARGSYY
DYDGFVYWGQGTLVTVSA).

Another aspect of the present application provides a method for preparing gene-edited CAR-T cells, comprising: disrupting the followings in the CAR-T cells by gene editing technology:
(i) A TRAC genomic region of chromosome 14 from position 23016448 to 23016490; (ii) A B2M genomic region from position 45003745 to 45003788 of chromosome 15.

In a specific embodiment, both the TRAC genomic region and the B2M genomic region are edited.

In a specific embodiment, the TRAC genomic region and the B2M genomic region are disrupted by homologous recombination, or gene editing technology of zinc finger nuclease, TALEN or CRISPR/Cas.

In yet another specific embodiment, the gene editing technology is CRISPR/Cas9 technology.

As used in this application, "CRISPR/Cas" is a gene editing technology that includes, but is not limited to, various naturally occurring or artificially designed CRISPR/Cas systems, such as the CRISPR/Cas9 system. The naturally occurring CRISPR/Cas system is an adaptive immune defense formed during the long-term evolution of bacteria and archaea, which can be used to fight invading viruses and foreign DNA. For example, CRISPR/Cas9 works by combining crRNA (CRISPR-derived RNA) with tracrRNA (trans-activating RNA) through base pairing to form a tracrRNA/crRNA complex, which guides the nuclease Cas9 protein to cleave double-stranded DNA at the sequence target site paired with the crRNA. By artificially designing, the tracrRNA and the crRNA can be engineered into an sgRNA (single guide RNA) with guiding effect, which is sufficient to guide a site-directed cleavage of DNA by Cas9. As an RNA-guided dsDNA-binding protein, the Cas9 effector nuclease can co-localize RNA, DNA, and protein, thus possessing great engineering potential. CRISPR/Cas systems can adopt class I, class II, or class III Cas proteins. In some embodiments of the application, the method uses Cas9. Other suitable CRISPR/Cas systems include, but are not limited to, systems and methods described in WO2013176772, WO2014065596, WO2014018423, and US 8,697,359.

In a specific embodiment, the present application provides a method for preparing a gene-edited CAR-T cell, comprising: disrupting the followings in the CAR-T cells by gene editing technology: (i) a target nucleotide sequence of the TRAC genomic region complementary to a sequence selected from any of SEQ ID NOs: 2 to 5; (ii) a target nucleotide sequence of the B2M genomic region complementary to a sequence selected from any of SEQ ID NOs: 6 to 13. Wherein, the TRAC genomic region and the B2M genomic region are disrupted by homologous recombination, or gene editing technology of zinc finger nuclease, TALEN or CRISPR/Cas.

In a specific embodiment, the present application provides a method for preparing gene-edited T cells, which disrupts the followings in the T cells by CRISPR/Cas9 gene editing technology:
(i) a target nucleotide sequence of the TRAC genomic region complementary to the sequence of SEQ ID NO: 2;
(ii) a target nucleotide sequence of the B2M genomic region complementary to the sequence of SEQ ID NO:8.

In a specific embodiment, the present application provides a method for preparing a gene-edited CAR-T cell, which disrupts the followings in the T cells by CRISPR/Cas9 gene editing technology:
(i) a target nucleotide sequence of the TRAC genomic region complementary to the sequence of SEQ ID NO: 2;
(ii) a target nucleotide sequence of the B2M genomic region complementary to the sequence of SEQ ID NO:8.

In a specific embodiment, the present application provides a method for preparing gene-edited T cells, which disrupts the followings in the CAR-T cells by CRISPR/Cas9 gene editing technology:
(i) a target nucleotide sequence of the TRAC genomic region complementary to the sequence of SEQ ID NO: 3;
(ii) a target nucleotide sequence of the B2M genomic region complementary to the sequence of SEQ ID NO:7.

Further, in a specific embodiment, the present application provides a method for preparing gene-edited CAR-T cells, comprising:
(i) introducing a guide RNA (sgRNA) comprising a sequence targeting a TRAC genomic region into a T cell to edit the TRAC genomic region; and/or
(ii) introducing a guide RNA (sgRNA) comprising a sequence targeting a B2M genomic region into the T cell to edit the B2M genomic region.

In a specific embodiment, the method comprises introducing nucleotide sequences encoding the sgRNA and Cas9 concurrently or separately into the T cells.

In the present application, "sgRNA (single guide RNA)" and "gRNA (guide RNA)" or may be "single guide RNA", "synthetic guide RNA" or "guide RNA" are used interchangeably. The sgRNA of the present application comprises a guide sequence that targets the sequence of interest.

In a specific embodiment, the sgRNA targets the gene encoding the constant region of the α and/or beta chain of TCR2, thereby disrupting the structure of the TCR on the surface of the CAR-T cell, rendering the molecule nonfunctional.

In a specific embodiment, the sgRNA targets the gene encoding β2 microglobulin (B2M), e.g., the first exon region of the gene encoding the B2M protein, thereby disrupting the structure of B2M, rendering the molecule nonfunctional.

Further, in a specific embodiment, a method for preparing a gene-edited CAR-T cell is provided, comprising: (i) introducing an sgRNA comprising a sequence selected from any of SEQ ID NOs: 2 to 5 into a T cell to edit a TRAC genomic region; (ii) introducing an sgRNA comprising a sequence selected from any of SEQ ID NOs: 6 to 13 into the CAR-T cell to edit a B2M genomic region. In some embodiments, the method comprises introducing the nucleotide sequences encoding the sgRNA and Cas9 concurrently or separately into the T cell.

In a specific embodiment of the present application, a method for preparing universal CAR-T cells is provided, comprising: (i) introducing TRAC-sg3 sgRNA into a T cell to edit a TRAC genomic region; (ii) introducing B2M-sg2 sgRNA into the T cell to edit a B2M genomic region. In some embodiments, the method comprises introducing the nucleotide sequences encoding the sgRNA and Cas9 concurrently or separately into the T cells.

In a specific embodiment of the present application, a method for preparing universal CAR-T cells is provided, comprising: (i) introducing an sgRNA comprising the sequence of SEQ ID NOs: 2 or 3 into CAR-T cells to edit a TRAC genomic region; (ii) introducing an sgRNA comprising a sequence selected from any of SEQ ID NOs: 7-11 into T cells to edit the B2M genomic region. In some embodiments, the method comprises introducing Cas9 or its encoding nucleotide into the CAR-T cell.

In some embodiments, the sgRNA described above is chemically modified. For example, 2'-O-methyl and/or internucleotide 3' thiol modifications. In some embodiments, the chemical modification is a 2'-O-methyl modification of the first one, two and/or three bases at the 5' end and/or the last base at the 3' end of the sgRNA.

In general, a guide sequence in an sgRNA is any polynucleotide sequence that has sufficient complementarity to a target polynucleotide sequence to hybridize to the target sequence and direct sequence-specific binding of the CRISPR complex to the target sequence. In some embodiments, when optimally aligned using an appropriate alignment algorithm, the degree of complementarity between the guide sequence and its corresponding target sequence is about or greater than about 80%, 85%, 90%, 95%, 97.5%, 99% or more. Optimal alignment can be determined using any suitable algorithm for aligning sequences, non-limiting examples of which include Smith-Waterman algorithm, Needleman-Wimsch algorithm, Burrows-Wheeler Transform based algorithms (e.g., Burrows Wheeler Aligner), ClustalW, Clutai X, BLAT, Novoalign (Novocraft Technologies), ELAND (Illumina, San Diego, CA), SOAP (available at soap.genomics.org.cn) and Maq (available at maq.sourceforge.net). In some embodiments, the guide sequence length can be about or greater than about 10, 11, 12, 13, 14, 15, 16, 17, 18, 19, 20, 21, 22, 23, 24, 25, 26, 27, 28, 29, 30, 35, 40, 45, 50, 55, 60, 65, 70, 75 or more nucleotides. In some embodiments, the guide sequence is less than about 75, 70, 65, 60, 55, 50, 45, 40, 35, 30, 25, 20, 15, 12 or fewer nucleotides in length. The ability of the guide sequence to direct sequence-specific binding of the CRISPR complex to the target sequence can be assessed by any suitable assay. For example, the components of a CRISPR system sufficient to form a CRISPR complex can be provided to a host cell with a corresponding target sequence, such as by transfection with a vector of the sequence encoding the CRISPR components, followed by the assessment of preferential cleavage within the target sequence. Likewise, cleavage of a target polynucleotide sequence can be then assessed in a test tube by providing the target sequence, components of a CRISPR complex (comprising the guide sequence to be tested and a control guide sequence different from the guide sequence), and comparing the binding or cleavage rates of the test and control guide sequences at the target sequence. The above assays and assessments can also be performed using other assays known to those skilled in the art.

In a specific embodiment, the sgRNA is concurrently introduced into the T cell with the nucleotide sequence encoding Cas9 mRNA by electroporation.

Specifically, the TRAC-targeting sgRNA, B2M-targeting sgRNA and/or Cas9-encoding nucleotides (e.g., mRNA) are introduced into the T cells by electroporation. In some embodiments, the TRAC-targeting sgRNA, B2M-targeting sgRNA, and Cas9-encoding nucleotides are concurrently introduced into the T cells by electroporation.

Specifically, the Cas9-encoding nucleotide is an mRNA, such as an mRNA comprising an ARCA cap. In some embodiments, the Cas9-encoding nucleotide is in a viral vector, such as a lentiviral vector. In some embodiments, the Cas9-encoding nucleotide comprises the sequence shown in SEQ ID NO:1. In some embodiments, the TRAC-targeting sgRNA, the B2M-targeting sgRNA and the Cas9-encoding nucleotides are in the same vector.

In some specific embodiments, the electroporation condition is any one selected from the group consisting of the following conditions: 150-250V, 0.5-2ms; 180-250V, 0.5-2ms; 150V, 2ms; 160V, 2ms; 170V, 2ms; 180V, 2ms; 190V, 1ms; 200V, 1ms; 210V, 1ms; 220V, 1ms; 230V, 1ms; 240V, 1ms; 250V, 0.5ms.

In some embodiments of the present application, the TRAC-targeting sgRNA and B2M-targeting sgRNA are concurrently introduced into the CAR-T cells. In a specific embodiment, when the TRAC-targeting sgRNA and the B2M-targeting sgRNA are concurrently introduced into the CAR-T cells, the amounts between the TRAC-targeting sgRNA and the B2M-targeting sgRNA can be similar or equivalent. In some embodiments, the TRAC-targeting sgRNA and the B2M-targeting sgRNA are introduced into the CAR-T cells one by one in any suitable order. In some embodiments, the TRAC-targeting sgRNA, the B2M-targeting sgRNA and the Cas9-encoding nucleotides are introduced into the CAR-T cell concurrently. In some embodiments, the Cas9-encoding nucleotide is introduced into the CAR-T cell prior to the TRAC-targeting sgRNA and the B2M-targeting sgRNA. In some embodiments, the CAR-T cell comprises a Cas9-encoding nucleotide or a Cas9 protein.

Further, the application provides a method for preparing genetically engineered CAR-T cells by CRISPR/Cas9 gene editing technology, comprising:
(i) introducing an sgRNA comprising any sequence selected from the group consisting of SEQ ID NOs: 2 to 5 into a CAR-T cell to edit a TRAC genomic region of chromosome 14 from position 23016448 to 23016490;
(ii) introducing an sgRNA comprising any sequence selected from the group consisting of SEQ ID NOs: 6 to 13 into CAR-T cells to edit a B2M genomic region of chromosome 15 from position 45003745 to 45003788.

In some embodiments, the present application provides a method for preparing genetically engineered CAR-T cells by CRISPR/Cas9 gene editing technology, comprising:
(i) introducing an sgRNA comprising a sequence selected from SEQ ID NO: 2 or SEQ ID NO: 3 into a CAR-T cell to edit a TRAC genomic region of chromosome 14 from position 23016448 to 23016490;
(ii) introducing an sgRNA comprising a sequence selected from SEQ ID NO: 7 or SEQ ID NO: 8 into a CAR-T cell to edit a B2M genomic region of chromosome 15 from position 45003745 to 45003788.

In some embodiments, the present application provides a method for preparing genetically engineered CAR-T cells by CRISPR/Cas9 gene editing technology, comprising:
(i) introducing an sgRNA comprising the sequence of SEQ ID NO: 2 into a CAR-T cell to edit a TRAC genomic region of chromosome 14 from position 23016448 to 23016490;
(ii) introducing an sgRNA comprising the sequence of SEQ ID NO: 8 into CAR-T cells to edit the B2M genomic region of chromosome 15 from position 45003745 to 45003788.

In some embodiments, the present application provides a method for preparing genetically engineered CAR-T cells by CRISPR/Cas9 gene editing technology, wherein:
(i) introducing an sgRNA comprising the sequence of SEQ ID NO: 3 into CAR-T cells to edit a TRAC genomic region of chromosome 14 from position 23016448 to 23016490;
(ii) introducing an sgRNA comprising the sequence of SEQ ID NO: 7 into CAR-T cells to edit the B2M genomic region of chromosome 15 from position 45003745 to 45003788.

In some embodiments, the TRAC-targeting sgRNA and the B2M-targeting sgRNA are introduced into the CAR-T cell concurrently. In some embodiments, the sgRNAs (including the TRAC-targeting sgRNA and the B2M-targeting sgRNA) are with 2'-O-methyl and/or internucleotide 3' thiol modifications. In some embodiments, the chemical modification is a 2'-O-methyl modifications at the first one, two and/or three bases of the 5' end of and/or the last base of the 3' end of the sgRNA (including TRAC-targeting sgRNA, B2M-targeting sgRNA).

In some embodiments, the above-mentioned sgRNAs (including TRAC-targeting sgRNAs, B2M-targeting sgRNAs) are introduced into the CAR-T cells by electroporation. In some embodiments, the above-mentioned sgRNAs (including TRAC-targeting sgRNA, B2M-targeting sgRNA) and Cas9-encoding nucleotides (such as mRNA) are concurrently introduced into the CAR-T cells by electroporation. In some embodiments, the electroporation conditions include any one selected from the group consisting of: 150-250V, 0.5-2ms; 150V, 2ms; 160V, 2ms; 170V, 2ms; 180V, 2ms; 190V, 1ms; 200V, 1ms; 210V, 1ms; 220V, 1ms; 230V, 1ms; 240V, 1ms; 250V, 0.5ms.

In some embodiments, it further includes screening CAR-T cells with low expression of TRAC and B2M from gene-edited CAR-T cells. For example, the expression levels of the TRAC and B2M in the gene-edited CAR-T cells are 2/10 of those in the non-gene-edited CAR-T cells.

In some embodiments, the TRAC and B2M are independently knocked out with an efficiency of more than 90%; and collectively knocked out with an efficiency of more than 75%. Furthermore, the knockout efficiency of the two genes collectively is above 80%. Compared with the prior art, the T cell gene knockout method of the present application obtains high gene knockout efficiency.

"Knockout efficiency" can be represented at the gene level as the INDEL efficiency of producing a gene knockout, or it can be represented at the cellular level as the percentage of cells in which the gene knockout causes the protein expressed by the gene to disappear or significantly decrease. In the present application, "knockout efficiency" refers to the knockout efficiency calculated based on the latter. Those skilled in the art can understand that high knockout efficiency can improve the harvest rate of cells of interest and reduce production costs and treatment costs. The full name of "Indel" used in this application is insertion/deletion, that is, insertion and deletion mutation.

In the method proposed in this application, gene-edited CAR-T cells are introduced with mRNAs expressing TCRα/β antibodies, and after an expression period of 24 to 48h, they specifically recognize and clear TCRα/β-positive T cells. High-purity dual-gene (TRAC and B2M) knockout CAR-T cells can be achieved in the culture process. Among the cell population obtained by the method, the ratio of TCR-negative cells is 98% or 99% or more. This method not only saves the cost of production time, but also saves the cost of manpower and material.

Another aspect of the present application also relates to a method for preparing universal CAR-T cells, comprising:
(i) introducing a chimeric antigen receptor (CAR) or its encoding nucleotide into an activated T cell;
   In some embodiments, the method further comprises introducing CAS9 or its encoding nucleotide into the activated T cell; and
(ii) introducing an sgRNA comprising a sequence targeting the TRAC genomic region of chromosome 14 from position 23016448 to 23016490 into the T cell to disrupt the TRAC genomic region; and/or
(iii) introducing an sgRNA comprising a sequence targeting the B2M genomic region of chromosome 15 from position 45003745 to 45003788 into the T cell to disrupt the B2M genomic region; and
(iv) introducing an mRNA targeting TCR α/β into the gene edited CAR-T cell to remove TCR α/β positive cells to obtain the final product, i.e., universal CAR-T cell, wherein the sequence of mRNA is SEQ ID NO:25: 
CAGAUCGUGCUGACCCAGAGCCCCGCCAUCAUGAGCGCCAGCCCCGGCGAG
AAGGUGACCAUGACCUGCAGCGCCACCAGCAGCGUGAGCUACAUGCACUGGUA
UCAGCAGAAGAGCGGCACCAGCCCCAAGCGCUGGAUCUACGACACCAGCAAGC
UGGCCAGCGGCGUGCCCGCCCGCUUCAGCGGCAGCGGCAGCGGCACCAGCTACA
GCCUGACCATCAGCAGCAUGGAGGCCGAGGACGCCGCCACCUACUACUGCCAGC
AGUGGAGCAGCAACCCCCUGACCUUCGGCGCCGGCACCAAGCUGGAGCUGAAG
GGUGGCGGUGGCUCGGGCGGUGGUGGGUCGGGUGGCGGCGGAUCUGAGGUGCA
GCUGCAGCAGAGCGGCCCCGAGCUGGTGAAGCCCGGCGCCAGCGUGAAGAUGA
GCUGCAAGGCCAGCGGCUACAAGUUCACCAGCUACGUGAUGCACUGGGUGAAG
CAGAAGCCCGGCCAGGGCCUGGAGUGGAUCGGCUACAUCAACCCCUACAACGA
CGUGACCAAGUACAACGAGAAGUUCAAGGGCAAGGCCACCCUGACCAGCGACA
AGAGCAGCAGCACCGCCUACAUGGAGCUGAGCAGCCUGACCAGCGAGGACAGC
GCCGUGCACUACUGCGCCCGCGGCAGCUACUACGACUACGACGGCUUCGUGUA
CUGGGGCCAGGGCACCCUGGUGACCGUGAGCGCC
 (wherein, the underlined part is
the linker sequence).

In some embodiments, the method for preparing universal CAR-T cells, comprises:
(i) introducing a nucleotide encoding a chimeric antigen receptor (CAR) into the genome of a human T cell to make a CAR-T cell;
   The method further comprises introducing a CAS9 or its encoding nucleotide into the T cell; and
(ii) introducing an sgRNA comprising any one of SEQ ID NOs: 2 to 5 into the T cell to edit a TRAC genomic region of chromosome 14 from position 23016448 to 23016490;
(iii) introducing an sgRNA comprising any one of SEQ ID NOs: 6 to 13 into the T cell to edit a B2M genomic region of chromosome 15 from position 45003745 to 45003788; and
(iv) introducing an mRNA targeting TCRα/β into the gene-edited CAR-T cell to remove TCRα/β-positive cells to obtain the final product, i.e., universal CAR-T cell.

In some embodiments, the method for preparing universal CAR-T cells comprises:
(i) introducing a nucleotide encoding a chimeric antigen receptor (CAR) into a human T cell genome;
   The method further comprises introducing a CAS9 or its encoding nucleotide into the T cell; and
(ii) introducing an sgRNA comprising any sequence selected from SEQ ID NO: 2 or SEQ ID NO: 3 into the CAR-T cell to edit a TRAC genomic region of chromosome 14 from position 23016448 to 23016490; and /or
(iii) introducing an sgRNA comprising any sequence of SEQ ID NO: 7 or SEQ ID NO: 8 into the CAR-T cell to edit a B2M genomic region of chromosome 15 from position 45003745 to 45003788; and
(iv) introducing an mRNA targeting TCRα/β into the gene-edited CAR-T cell to remove TCRα/β-positive cells to obtain the final product, i.e., universal CAR-T cell.

In some embodiments, the method for preparing universal CAR-T cells comprises:
(i) introducing a nucleotide encoding a chimeric antigen receptor (CAR) into a human T cell genome;
   The method further comprises introducing a Cas9 or its encoding nucleotide into the T cell; meanwhile
(ii) introducing an sgRNA comprising the sequence of SEQ ID NO: 2 into the T cell to edit the TRAC genomic region of chromosome 14 from position 23016448 to 23016490;
(iii) introducing an sgRNA comprising the sequence of SEQ ID NO: 8 into the T cells to edit the B2M genomic region of chromosome 15 from position 45003745 to 45003788; and
(iv) introducing an mRNA targeting TCRα/β into the gene-edited CAR-T cell to remove TCRα/β-positive cells to obtain the final product, i.e., universal CAR-T cell.

In some embodiments, the method of preparing CAR-T cells comprises:
(i) introducing a nucleotide encoding a chimeric antigen receptor (CAR) into a T cell genome;
   The method further comprises introducing a CAS9 or its encoding nucleotide into the T cell; and
(ii) introducing an sgRNA comprising the sequence of SEQ ID NO: 3 into the T cell to edit the TRAC genomic region of chromosome 14 from position 23016448 to 23016490;
(iii) introducing an sgRNA comprising the sequence of SEQ ID NO: 7 into the T cell to edit the B2M genomic region of chromosome 15 from position 45003745 to 45003788; and
(iv) introducing an mRNA targeting TCRα/β into the gene-edited CAR-T cell to remove TCRα/β-positive cells to obtain the final product, i.e., universal CAR-T cell.

In some embodiments, the nucleotide encoding a chimeric antigen receptor (CAR) is introduced into the activated T cell. At the same time, the sgRNA targeting TRAC and the sgRNA targeting B2M are introduced into the above CAR-T. In some embodiments, the sgRNAs (including the sgRNA targeting TRAC and the sgRNA targeting B2M) are with 2'-O-methyl and/or internucleotide 3'-thiol modification. In some embodiments, the chemical modification is a 2'-O-methyl modification at the first one, two and/or three bases of the 5' end of and/or the last base of the 3' end of the sgRNAs (including the sgRNA targeting TRAC and the sgRNA targeting B2M).

In some embodiments, a nucleotide encoding CAR is introduced into the T cell genome to activate the T cell.

In some embodiments, the above-mentioned sgRNAs (including the sgRNA targeting TRAC and the sgRNA targeting B2M) are introduced into the CAR-T cell by electroporation. In some embodiments, the above-mentioned sgRNAs (including the sgRNA targeting TRAC and the sgRNA targeting B2M) and the nucleotide encoding Cas9 (e.g., mRNAs) are concurrently introduced into the T cells by electroporation. In some embodiments, the electroporation conditions include any one selected from the group consisting of: 150-250V, 0.5-2ms; 180-250V, 0.5-2ms; 150V, 2ms; 160V, 2ms; 170V, 2ms; 180V, 2ms; 190V, 1ms; 200V, 1ms; 210V, 1ms; 220V, 1ms; 230V, 1ms; or 240V, 1ms; 250V, 0.5ms.

In some embodiments, the mRNA targeting TCRα/β is introduced into the gene-edited CAR-T cell to remove TCRα/β-positive cells.

In one aspect, the present application relates to the universal CAR-T cells prepared by the above method.

In one aspect, the application relates to the CAR-T cells, comprising the above-described genetically engineered T cells expressing a chimeric antigen receptor (CAR).

In one aspect, the application relates to the CAR-T cells, in the CAR-T cells:
(i) The TRAC genomic region of chromosome 14 from position 23016448 to 23016490 is edited;
(ii) The B2M genomic region of chromosome 15 from position 45003745 to 45003788 is edited.

The location information of the TRAC genomic region and the B2M genomic region mentioned above in the present application is determined according to the wild sequence location information of the gene in the reference database: GRCh37 (hg19). Those skilled in the art know how to obtain the corresponding position information of the above-mentioned genomic regions with reference to other databases.

In one aspect, the present application also relates to compositions (such as pharmaceutical compositions), kits, and medical products comprising the above-mentioned genetically engineered T cells, or CAR-T cells.

In one aspect, the present application relates to a method of treating a disease in a subject, comprising administering to the subject an effective amount of the above-mentioned CAR-T cells. In some embodiments, the disease is tumor. In some embodiments, the tumor is hematological tumor. In some embodiments, the tumor lymphoma or leukemia.

In one aspect, the present application relates to a method for purifying gene-edited T cells and/or CAR-T cells to obtain 99% TCRα/β-negative universal CAR-T cells. In some embodiments, the protein expressed by translating the mRNA that targets TCRα/β and has the effect of clearing TCRα/β-positive cells is:

QIVLTQSPAIMSASPGEKVTMTCSATSSVSYMHWYQQKSGTSPKRWIYDTSKLAS
GVPARFSGSGSGTSYSLTISSMEAEDAATYYCQQWSSNPLTFGAGTKLELK ;

EVQLQQSGPELVKPGASVKMSCKASGYKFTSYVMHWVKQKPGQGLEWIGYINPYN
DVTKYNEKFKGKATLTSDKSSSTAYMELSSLTSEDSAVHYCARGSYYDYDGFVYWG
QGTLVTVSA;

The linker sequence is: GGGGSGGGGSGGGGSGGGGS (SEQ ID NO: 21).

In one aspect, the present application relates to an sgRNA or a vector thereof comprising any one of SEQ ID NOs: 2 to 13. In some embodiments, the sgRNA is chemically modified, such as a 2'-O-methyl and/or an internucleotide 3' thiol modification. In some embodiments, the chemical modification is a 2'-O-methyl modification at the first one, two and/or three bases at the 5' end and/or the last base at the 3' end of the sgRNA.

In some embodiments, the TCR and/or HLA genes of the universal CAR-T cells of the present application are knocked out.

In some specific embodiments, the α chain constant coding region (i.e., TRAC) gene of the TCR is knocked out. The coding region of B2M is knocked out.

In some specific embodiments, the α chain constant coding region (i.e., TRAC) gene of the TCR is knocked out. For example, in a specific embodiment, the TCR α chain constant coding region gene of the present application is knocked out by one of the TRAC-sg 2, 3, 4, 6 molecules (see Table 1) and the Cas 9 molecule introduced into the cell. Preferably, the α chain constant coding region gene of the TCR is knocked out by TRAC-sg 2 and a Cas9 molecule or TRAC-sg 3 and a Cas9 molecule introduced into the cell.

**Table 1**

| | | |
|---|---|---|
| T2 | GCUGGUACACGGCAGGGUCA | SEQ ID NO: 2 |
| T3 | CUCUCAGCUGGUACACGGCA | SEQ ID NO: 3 |
| T4 | AUUUGUUUGAGAAUCAAAAU | SEQ ID NO: 4 |
| T6 | UCUCUCAGCUGGUACACGGC | SEQ ID NO: 5 |
| B1 | ACUCUCUCUUUCUGGCCUGG | SEQ ID NO: 6 |
| B2 | GAGUAGCGCGAGCACAGCUA | SEQ ID NO: 7 |
| B3 | CGCGAGCACAGCUAAGGCCA | SEQ ID NO: 8 |
| B4 | UCACGUCAUCCAGCAGAGAA | SEQ ID NO: 9 |
| B5 | GCUACUCUCUCUUUCUGGCC | SEQ ID NO: 10 |
| B6 | UUUGACUUUCCAUUCUCUGC | SEQ ID NO: 11 |
| B7 | CGUGAGUAAACCUGAAUCUU | SEQ ID NO: 12 |
| B8 | CUCGCGCUACUCUCUCUUUC | SEQ ID NO: 13 |

In other specific embodiments, the B2M gene in the constant coding region of the HLA is knocked out. For example, in a specific embodiment, the B2M constant coding region gene of the present application is knocked out by one of the B2M-sg 1-8 molecules (see Table 1) and the Cas 9 molecule introduced into the cell, preferably B2M-sg 2 or B2M-sg 6 molecule, and the Cas9 molecule are introduced into the cell.

In other specific embodiments, the α chain constant coding region (i.e., TRAC) gene of the TCR and the B2M gene are knocked out. For example, in a specific embodiment, the TRAC and B2M genes of the application are knocked out by the TRAC-sg3 and B2M-sg2 molecules (see Table 1) and the Cas 9 molecule introduced into the cell.

In some embodiments, the present application provides a method for efficiently editing CAR-T cells, comprising the following steps:
Introducing sgRNA molecules and Cas9 molecules into the CAR-T cells:
In some embodiments, the sgRNA molecule comprises a targeting domain complementary to a target region in the α chain constant coding region (i.e., TRAC) gene of the TCR, or the constant coding region gene of the B2M gene.

In some embodiments, the sgRNA molecule refers to a nucleic acid sequence comprising a targeting domain complementary to the target region of the gene to be knocked out, which can recognize the target DNA sequence and guide the Cas9 molecule to cleave the target site, which can knock out the corresponding sites in one step with high efficiency (knockout efficiency of more than 85%).

In some embodiments, the sequence of the targeting domain comprised by the sgRNA molecule is one of those shown in Table 1.

In some preferred embodiments, the sequence of the targeting domain is shown in T2 or B3.

In some preferred embodiments, the sgRNA molecule and the mRNA encoding the Cas9 molecule are introduced into the CAR-T cell by electroporation.

In some specific embodiments, the T cells used in the above methods are derived from healthy humans, e.g., peripheral blood of healthy adults, or umbilical cord blood of healthy humans who gave birth spontaneously.

In some embodiments, the third aspect of the present application provides sgRNA sequences with high editing efficiency after specific modifications (Table 1).

In some specific embodiments, the sgRNA is synthesized and modified by chemical methods, so that the sgRNA has more stable and higher editing efficiency than that of the sgRNA obtained by ordinary *in vitro* transcription (IVT). Preferably, the CAR-T cells are electroporated once, and the gene editing efficiency of the chemically synthesized and modified sgRNA is more than 10 times that of the sgRNA obtained by ordinary IVT.

In some embodiments, the mRNAs targeting TCRα/β obtained by common *in vitro* transcription (IVT) method are delivered to the gene-edited CAR-T cells by the method of electroporation delivery. With the increase of culture time, TCRα/β-negative universal CAR-T cells are obtained at the time of harvest.

In some embodiments, the use of the universal CAR-T cells of the present application for preparing a medicament for treating a disease (such as a tumor) is provided.

In some specific embodiments, the α chain constant coding region (i.e., TRAC) gene of the TCR, the constant coding region B2M gene of HLA. For example, in a specific embodiment, the TCRα chain constant coding region gene of the present application is knocked out by the TRAC-sg 2 molecule and the Cas9 molecule introduced into the cell, and the B2M constant coding region gene of the present application is knocked out by the B2M-sg6 molecule and the Cas 9 molecule introduced into the cell: preferably, the α chain constant coding region gene of the TCR is knocked out by TRAC-sg2 and the Cas9 molecule introduced into the cell, and the B2M constant coding region gene is knocked out by the B2M-sg6 and the Cas9 molecule introduced into the cell.

In another aspect of the present application, a method for preparing CAR-T cells (such as universal CAR-T cells) is also provided. In some embodiments, the method comprises introducing a CAR or its encoding nucleotide or vector into any of the genetically engineered T cells described herein (e.g., universal T cells).

In some embodiments, a method of preparing CAR-T cells is provided, comprising:
(i) introducing a chimeric antigen receptor (CAR) or nucleic acid encoding it into the T cell; and
(ii) introducing an sgRNA comprising a sequence targeting a TRAC genomic region of chromosome 14 from position 23016448 to 23016490 into the T cell to disrupt the TRAC genomic region; and
(iii) introducing an sgRNA comprising a sequence targeting a B2M genomic region of chromosome 15 from position 45003745 to 45003788 into the T cell to disrupt the B2M genomic region; and
(iv) introducing an sgRNA comprising a sequence targeting a PD-1 genomic region of chromosome 2 from position 242800936 to 242800978, or a PD-1 genomic region of chromosome 2 from position 242795009 to 242795051 into the T cell to disrupt the PD-1 genomic region;
(v) introducing an mRNA targeting TCRα/β into the gene-edited CAR-T cell to remove TCRα/β-positive cells to obtain the final product, i.e., universal CAR-T cell.

CAR or its encoding nucleotide and the sgRNA targeting TRAC, the sgRNA targeting B2M and CAR or its encoding nucleotide can be introduced into T cells in any suitable order, but the TCRα/β-targeting mRNA must be introduced into the gene-edited CAR -T cell, so as to obtain the final product, i.e., universal CAR-T cell. In some embodiments, the sgRNA targeting TRAC, the sgRNA targeting B2M, and CAR or its encoding nucleotide are concurrently introduced into the T cell, and then, the TCRα/β-targeting mRNA is introduced into the gene-edited CAR-T cell. In some embodiments, the CAR or its encoding nucleotide is introduced into the T cell before the sgRNA targeting TRAC, the sgRNA targeting B2M, and then, and the TCRα/β-targeting mRNA is introduced into the gene-edited CAR-T cell. In some embodiments, the CAR or its encoding nucleotide is introduced into the gene-edited T cell, the TRAC and B2M genomic regions of which have been disrupted by gene editing. In some embodiments, the method further comprises introducing Cas9 or its encoding nucleotide together with the sgRNA into the T cell, and then introducing the mRNA targeting TCRα/β into the gene-edited CAR-T cell.

In some embodiments, the CAR expressed by the universal CAR-T cell of the present application can be any CAR that targets cancerous cells other than TCRα/β, thereby exerting a killing effect.

In some embodiments, the CAR expressed in the CAR-T cell of the present application comprises sequentially linked signal peptide, extracellular binding region, hinge region, transmembrane region and intracellular signal region. The term "signal peptide" as used herein refers to a short (e.g., 5-30 amino acids in length) peptide chain that directs the transfer of newly synthesized proteins to the secretory pathway. In the present application, the signal peptide of various proteins in the human body, such as a signal peptide of a cytokine protein or a leukocyte differentiation antigen (CD molecule) secreted *in vivo* can be used.

In some embodiments, the signal peptide is a CD8 signal peptide, e.g., its amino acid sequence is as shown in the invention patent application US20140271635A1.

In some embodiments, the hinge region may use hinge regions of various antibodies or antigen receptors, particularly the hinge region of CD molecule. In a specific embodiment, the hinge region may be selected from the hinge regions of proteins such as CD8 or CD28. The CD8 or CD28 is a natural marker on the surface of a T cell.

In the present application, the transmembrane regions of various human proteins, especially the transmembrane regions of various different antigen receptors, can be used. The transmembrane region preferably used is the transmembrane region of a CD molecule. In one embodiment, the transmembrane region is selected from the transmembrane region of a CD8 protein.

In some embodiments, the hinge region is a CD8α hinge region (CD8-hinge), the amino acid sequence of which is as shown in invention patent application US20140271635A1.

The "extracellular binding region" refers to the region comprising the region specifically recognizing a target antigen. In some embodiments, the extracellular binding region comprises a region that specifically recognizes a target tumor cell surface antigen. For example, this region may be an antigen-binding fragment of an scFv or other antibody. The term "scFv" as used herein refers to a recombinant protein of a heavy chain variable region (VH) and a light chain variable region (VL) connected by a linker, the linker links the two domains, ultimately forming the antigen-binding site. A scFv is usually an amino acid sequence encoded by a chain of a nucleotide. The above-mentioned scFv may also include derivatives thereof.

The CAR used in the present application and each of its domains can be further modified by using conventional techniques known in the art, such as amino acid deletion, insertion, substitution, addition, and/or recombination, and/or other modification methods, alone or in combination. Methods for introducing such modifications into the DNA sequence of an antibody according to its amino acid sequence are well known to those skilled in the art (see, e.g., Sambrook Molecular Cloning: A Laboratory Manual, Cold Spring Harbor Laboratory (1989) N.Y). The modifications are preferably performed at the nucleic acid level.

The term "specific recognition" as used herein means that the antigen recognition region of the present application does not or substantially does not cross-react with any polypeptide other than the target antigen. The degree of its specificity can be judged by immunological techniques, including but not limited to, immunoblotting, immunoaffinity chromatography, flow cytometry, and the like.

In some embodiments, the extracellular binding region is an antigen binding region that specifically recognizes a target other than TCRα/β.

In the present application, the intracellular signal region is the intracellular signal region of a CD137(4-1BB) protein. The CD3 molecule consists of five subunits, of which the CD3ζ subunit (also known as CD3zeta, ζ for short) comprises three ITAM motifs, which are important signal transduction regions in the TCR-CD3 complex. FcεRI y is mainly distributed on the surface of mast cells and basophils, and it comprises an ITAM motif similar to CD3ζ in structure, distribution and function. In addition, as mentioned above, CD137 is a costimulatory signaling molecule, and the costimulatory effect of its intracellular signaling segment after binding to the respective ligands causes continuous proliferation of T cells, and can increase the level of the secretion of cytokines, such as IL-2 and IFN-γ, by T cells, while improving the survival period and anti-tumor effect of the CAR-T cells *in vivo.*

In the present application, the mRNA sequence targeting TCRα/β obtained by *in vitro* transcription (IVT) is delivered to the gene-edited CAR-T cell through electroporation, and the protein targeting TCRα/β is expressed through *in vivo* translation. As it is a transiently transfected, the protein can only exist for 4-5 days, and will disappear after its function of clearing TCRα/β T cells is completed. It is guaranteed that the final product is pure universal CAR-T.

In certain embodiments, the signal generated by CAR alone is not sufficient to fully activate a naive T cell, and sequences for initiation of antigen-dependent primary activation by the TCR (primary intracellular signaling domain) and sequences acts in an antigen-independent manner to provide a costimulatory signal (costimulatory domain) are required. A primary signaling domain regulates primary activation of a TCR complex in a stimulatory or inhibitory manner. A primary intracellular signaling domain that act in a stimulatory manner may comprise signaling motifs known as immunoreceptor tyrosine activation motifs (ITAMs). The primary Intracellular signaling sequence comprising ITAM in the present application is CD3ζ. In one embodiment, the primary signaling domain comprises a modified ITAM domain, e.g., a mutated ITAM domain whose activity is altered (e.g., increased or decreased) compared to the native ITAM domain, or a truncated ITAM primary Intracellular signaling domain. In one embodiment, the primary signaling domain comprises one or more ITAM motifs.

A costimulatory signaling domain refers to the portion of a TCR that comprises the intracellular domain of a costimulatory molecule. Costimulatory molecules are cell surface molecules other than antigen receptors or their ligands that are required for efficient lymphocyte responses to antigens. The costimulatory molecule region of the present application is 4-1BB (CD137).

In some embodiments, the present application provides a method for preparing CAR-T cells, such as universal CAR-T cells, comprising the following steps:
1) introducing a CAR molecule into a T cell;
   In some embodiments, the sgRNA molecule refers to a nucleic acid sequence comprising a targeting domain complementary to a target region of a gene to be knocked out, which can recognize the target DNA sequence and guide the Cas9 molecule to cleave the target site, achieving high-efficiency of knockout (more than 85% knockout efficiency) of the corresponding site in one step.
2) introducing sgRNA molecules and Cas9 molecules into the CAR-T cell:
   In some embodiments, the sgRNA molecules comprise targeting domains complementary to a target region of an α chain constant coding region (i.e., TRAC) gene of TCR, a constant coding region of B2M gene of HLA, and a constant coding region gene of PD-1.
3) introducing an mRNA molecule targeting TCRα/β into the CAR-T cell;

In some embodiments, the mRNA molecule targeting TCRα/β is a molecule transcribed *in vitro,* which can be translated into protein in cells and expressed on the cell membrane to remove TCRα/β-positive T cells.

In some embodiments, the Cas9 molecule refers to a Cas9 mRNA, which is capable of cleaving a target site under the guidance of sgRNA.

In some specific embodiments, the sequence of the targeting domain comprised in the sgRNA molecule is as shown in any one of Table 1.

In some preferred embodiments, the sequence of the targeting domain is shown in T2 or B3 (Table 1).

In some preferred embodiments, the sgRNA molecule and the mRNA encoding the Cas9 molecule are introduced into the T cell by electroporation.

In some embodiments, the CAR molecule is introduced into the T cell by, for example, lentiviral transfection techniques.

In some specific embodiments, the step of isolating and/or activating T cells from healthy human peripheral blood or umbilical cord blood is included; preferably, the method further includes the step of sorting the universal CAR-T cells after the above step 2); after performing the above step 3), functional verification on the obtained CAR-T cells, such as the universal CAR-T cells is performed.

In some embodiments, the present application provides the use of the above-mentioned CAR-T cells for preparing a medicament for the treatment of a disease (such as tumor).

In one aspect, the present application provides a method for treating a disease in a subject, comprising administering to the subject an effective amount of the CAR-T cells of the present application. Diseases described in the present application include but are not limited to cancer and HIV/AIDS. In some embodiments, the disease is tumor, including a hematological tumor such as lymphoma or leukemia. In some embodiments, the CAR can target other cancer cell targets other than TCRα/β antigens. In some embodiments, the T cells are not obtained from a subject. For example, the T cells can be derived from healthy donors.

The CAR-T cells involved in the present application can be administered to a subject in need thereof by a route conventionally used for the administration of pharmaceutical products comprising cell components, such as an intravenous infusion route. The dose to be administered can be specifically determined according to the condition and general health state of the subject.

The present application also relates to sources of T cells. The T cells are obtained from the subject prior to expansion and genetic modification. The term "subject" is intended to include living organisms (e.g., mammals) capable of eliciting an immune response. Examples of subjects include humans. The T cells can be obtained from a variety of sources, including peripheral blood mononuclear cells, bone marrow, lymph node tissue, cord blood, thymus tissue, tissue from sites of infection, ascites, pleural effusion, spleen tissue, and tumors. The T cells of the present application can also be derived from hematopoietic stem cells at various stages of differentiation. In directional differentiation culture conditions, hematopoietic stem cells differentiate into T cells. In certain aspects of the present application, various T cell lines available in the art can be used.

In certain aspects of the application, T cells can be obtained from blood collected from a subject using a variety of techniques known to those skilled in the art, such as Ficoll^{™} isolation. Cells can also be obtained from an individual's circulating blood by apheresis. Apheresis products typically comprise lymphocytes, including T cells, monocytes, granulocytes, B cells, other nucleated leukocytes, red blood cells, and platelets. In one aspect, cells collected by apheresis can be washed to remove the plasma fraction and placed in an appropriate buffer or medium for subsequent processing steps.

T cells can be isolated from peripheral blood lymphocytes by lysing red blood cells and depleting monocytes by, e.g., PERCOLL^{™} gradient centrifugation or counterflow centrifugation. Specific T cell subpopulations such as CD3+, CD28+, CD4+, CD8+, CD45RA+ and CD45RO+ T cells can be further isolated by positive or negative selection techniques. For example, in one aspect, T cells are isolated by incubating with anti-CD3/anti-CD28 (e.g., 3x28) conjugated beads, such as DYNABEADS^{™} M-450CD3/CD28T for a period of time sufficient to positively select desired T cells. Tumor infiltrating lymphocytes (TILs) can be isolated from tumor tissues.

In one aspect of the present application, an sgRNA targeting TRAC and an sgRNA targeting B2M are also provided. The sgRNA comprises any nucleotide sequence selected from SEQ ID NOs: 2-22. In some embodiments, the sgRNA is chemically modified.

The present application also includes sgRNA compositions, kits or articles of manufacture, which include the sgRNA involved in the present application or the vector thereof. In some embodiments, the kit comprises: i) the sgRNA comprising a sequence selected from any one of SEQ ID NOs: 2 to 5; (ii) the sgRNA comprising a sequence selected from any one of SEQ ID NOs: 6 to 13; and/or (iii) the sgRNA comprising a sequence selected from any one of SEQ ID NOs: 15-22. In some embodiments, the kit comprises: (i) the sgRNA comprising the sequence of SEQ ID NO:3; (ii) the sgRNA comprising the sequence of SEQ ID NO:16; and (iii) the sgRNA comprising the sequence of SEQ ID NO: 7. In some embodiments, the kit further includes a Cas6-encoding nucleic acid or a vector thereof. In some embodiments, the sgRNA is chemically modified.

In some embodiments, the genetic modification method of the T cells in the present application uses chemically modified sgRNAs. The chemically modified sgRNA adopted by the present inventors is believed to have the following two advantages. First, since sgRNA is a single-stranded form of RNA, its half-life is very short. After entering the cell, it will be rapidly degraded (up to 12 hours), while it takes at least 48hrs for a Cas9 protein to bind sgRNA for gene editing. Therefore, using a chemically modified sgRNA, after entering a cell, its expression is stable, and it binds to the Cas9 protein to efficiently edit the genome and generate Indels. Second, an unmodified sgRNA has poor ability to penetrate cell membrane and cannot effectively enter a cell or tissue to perform corresponding function. The ability of a chemically modified sgRNA to penetrate cell membrane is usually enhanced. In the present application, chemical modification methods commonly used in the art can be used, as long as the stability of the sgRNA can be enhanced (extending the half-life) and the ability to enter the cell membrane can be improved, any of these methods can be used. In addition to the specific chemical modifications used in the examples, other modification methods are also included, for example, the chemical modification method reported in the literature: Deleavey GF1, Damha MJ. Designing chemically modified oligonucleotides for targeted gene silencing. Chem Biol. 2012 Aug 24;19(8):937-54, and Hendel et al. Chemically modified guide RNAs enhance CRISPR-Cas genome editing in human primary cells. Nat Biotechnol. 2015 Sep;33(9):985-989.

In the present application, chemically modified sgRNA and Cas9-encoding gene are concurrently electroporated into T cells, resulting in high gene editing efficiency (e.g., represented as Indels%), wherein chemical modification of sgRNA is one of the key factors in the present application. The data in the examples show that if the chemically unmodified sgRNA was electroporated with Cas9 mRNA, the Indels efficiency was much lower than that obtained when the chemically modified sgRNA was electroporated.

The content of the present application will be described below through specific embodiments. It should be understood that the specific embodiments are only for illustrative purposes, and do not mean that the content of the present application is limited to the specific embodiments.

Several documents are cited throughout the text of this specification. Each document herein (including any journal articles or abstracts, published or unpublished patent applications, issued patents, manufacturer's instructions, instructions for use, etc.) is hereby incorporated by reference. However, there is no admission that the documents cited herein are in fact prior art to the present application.

### Example

The content of the present application will be described below with reference to specific examples, but the scope of the present application is not limited thereto.

Unless otherwise specified, the reagents and devices used in the following examples are conventional reagents and devices in the art, and can be obtained commercially. The methods used herein are all routine experimental methods, and those skilled in the art can implement the described solutions and obtain corresponding results without any doubt according to the content of the examples.

### Example 1: Preparation of universal CAR-T cells

Preparation and expansion of universal CAR-T cells

The sorted T cells were activated with cytokines, and then the cell density was adjusted to 1 × 10⁶ cells/mL with T cell culture medium. After 24h, the virus comprising CAR (extracellular region CD19 scFv, linker, CD8 α hinge region, CD8 transmembrane domain, 4-11BB signaling domain) and CD3 zeta, the specific structure is shown in US20140271635A1, the specific sequence is shown in Table 2) was used to infect T cells at MOI=4; after 72h, observe the state of the cells, collect the cell suspension, centrifuge it at 300g for 7min, discard the supernatant, and wash it twice with DPBS solution (manufacturer: Gibco; Cat. NO.: 1924294), and then adjust the cell density to 2.5×10⁷ cells/mL with electroporation reagent medium.

**Table 2**

| name | sequence |
|---|---|
| CD19 light chain coding DNA | Gacatccagatgacacagactacatcctccctgtctgcctctctgggagacagagtcaccatcagttgcagggcaa gtcaggacattagtaaatatttaaattggtatcagcagaaaccagatggaactgttaaactcctgatctaccatacatca agattacactcaggagtcccatcaaggttcagtggcagtgggtctggaacagattattctctcaccattagcaacctg gagcaagaagatattgccacttacttttgccaacagggtaatacgcttccgtacacgttcggaggggggaccaagct ggagatcaca(SEQ ID No：22) |
| Linker region coding DNA | ggtggcggtggctcgggcggtggtgggtcgggtggcggcggatct(SEQ ID No: 23) |
| CD19 heavy chain coding DNA | gaggtgaaactgcaggagtcaggacctggcctggtggcgccctcacagagcctgtccgtcacatgcactgtctca ggggtctcattacccgactatggtgtaagctggattcgccagcctccacgaaagggtctggagtggctgggagtaat atgggggtagtgaaaccacatactataattcagctctcaaatccagactgaccatcatcaaggacaactccaagagcc aagttttcttaaaaatgaacagtctgcaaactgatgacacagccatttactactgtgccaaacattattactacggtggt agctatgctatggactactggggccaaggaacctcagtcaccgtctcctca(SEQ ID No：24) Accacgacgccagcgccgcgaccaccaacaccggcgcccaccatcgcgtcgcagcccctgtccctgcgccca gaggcgtgccggccagcggcgggggcgcagtgcacacgagggggctggacttcgcctgtgat（SEQ ID No：15） |
| CD8 α Hinge region | Accacgacgccagcgccgcgaccaccaacaccggcgcccaccatcgcgtcgcagcccctgtccctgcgccca gaggcgtgccggccagcggcgggggcgcagtgcacacgagggggctggacttcgcctgtgat（SEQ ID No：15） |
| CD8 Transmembran e region | atctacatctgggcgcccttggccgggacttgtggggtccttctcctgtcactggttatcaccctttactgc ( SEQ ID No: 16) |
| 4-11BB signaling region | Aaacggggcagaaagaaactcctgtatatattcaaacaaccatttatgagaccagtacaaactactcaagaggaag atggctgtagctgccgatttccagaagaagaagaaggaggatgtgaactg（SEQ ID No：17） |
| CD3 zeta | Agagtgaagttcagcaggagcgcagacgcccccgcgtacaagcagggccagaaccagctctataacgagctca atctaggacgaagagaggagtacgatgttttggacaagagacgtggccgggaccctgagatgggggggaaagcc gagaaggaagaaccctcaggaaggcctgtacaatgaactgcagaaagataagatggcggaggcctacagtgag attgggatgaaaggcgagcgccggaggggcaaggggcacgatggcctttaccagggtctcagtacagccacca aggacacctacgacgcccttcacatgcaggccctgccccctcgc（SEQ ID NO：18） |

Cas9 mRNA prepared using HISCRIBE^{™} T7 ARCA mRNA Kit (tailed) (manufacturer: NEB, catalog number: cat#E2060S) and synthesized sgRNAs (sgRNA targeting TRAC sequence: GCUGGUACACGGCAGGGUCA; sgRNA targeting B2M sequence: GAGUAGCGCGAGCACAGCUA) were used. T cells and the RNA were mixed to make the final concentration to be 2.5×10⁶ cells and 8µg RNA (4µg of Cas9 mRNA and each sgRNA) per 100µL, and then the RNA was introduced into the cells by electroporator BTX Agile pulse MAX and cultured. On day 5 of culture, the CAR-T cells were mixed with the target TCRα/β mRNA 
CAGAUCGUGCUGACCCAGAGCCCCGCCAUCAUGAGCGCCAGCCCCGGCGAG
AAGGUGACCAUGACCUGCAGCGCCACCAGCAGCGUGAGCUACAUGCACUGGUA
UCAGCAGAAGAGCGGCACCAGCCCCAAGCGCUGGAUCUACGACACCAGCAAGC
UGGCCAGCGGCGUGCCCGCCCGCUUCAGCGGCAGCGGCAGCGGCACCAGCTACA
GCCUGACCATCAGCAGCAUGGAGGCCGAGGACGCCGCCACCUACUACUGCCAGC
AGUGGAGCAGCAACCCCCUGACCUUCGGCGCCGGCACCAAGCUGGAGCUGAAG
GGUGGCGGUGGCUCGGGCGGUGGUGGGUCGGGUGGCGGCGGAUCUGAGGUGCA
GCUGCAGCAGAGCGGCCCCGAGCUGGTGAAGCCCGGCGCCAGCGUGAAGAUGA
GCUGCAAGGCCAGCGGCUACAAGUUCACCAGCUACGUGAUGCACUGGGUGAAG
CAGAAGCCCGGCCAGGGCCUGGAGUGGAUCGGCUACAUCAACCCCUACAACGA
CGUGACCAAGUACAACGAGAAGUUCAAGGGCAAGGCCACCCUGACCAGCGACA
AGAGCAGCAGCACCGCCUACAUGGAGCUGAGCAGCCUGACCAGCGAGGACAGC
GCCGUGCACUACUGCGCCCGCGGCAGCUACUACGACUACGACGGCUUCGUGUA
CUGGGGCCAGGGCACCCUGGUGACCGUGAGCGCC
 (the underlined part is the linker
sequence) prepared by the HISCRIBE^{™} T7 ARCA mRNA Kit (tailed) (manufacturer: NEB, catalog number: cat#E2060S) to make the final concentration reach 2×10⁷ cells per 200uL, and then the RNA was introduced into the cells using the electroporator BTX Agile pulse MAX. Then the cells and the cells without the electroporation with the mRNA were mixed and cultured at a ratio of 1:2. The growth status of the cells was observed every day, and the cell counts were performed every other day. As shown in Figure 1, the viability of the cells in the process of expansion culture was monitored. After adding the mRNA targeting TCRα/β, the cell viability was not good in the later stage, because the cells could kill TCRα/β-positive T cells by themselves, but with the increase of culture time, the cell viability had no significant difference with the other two groups. Figure 2 shows the fold expansion of cells after 14 days of culture. Due to the self-killing of the CAR-added cells, the expansion ratio was not as good as that of other control groups.

Figure 3 shows the phenotypic analysis of the gene-edited CAR-T cells cultured after the introduction of TCRα/β-targeted CAR mRNA. It can be seen from the results that after the DKO-T cells added with TCRα/β-CAR were cultured, the proportion of TCRα/β negative cells was no less than 99%.

Figure 4 shows the positive proportion changes of TCRα/β after MIX (DKO CAR-T:DKO CAR-T+mRNA 1:1) was cultured. After 2 days of mixed culture, the proportion of negative TCRα/β cells was no less than 99%.

Figure 5 shows the purification recovery rate of TRAC/B2M (DKO CAR-T) knockout T cells after screening and purification with STEMCELL. It can be seen from the figure that the recovery rate was low, and the recovery rate was basically between 20% and 30%.

In this example, the TCR positivity rate was 1%, and the T cell positivity rate of TCR and B2M DKO was <1%.

### Example 2: Functional Verification of Universal CAR-T

The killing effect of the universal CAR-T cells (i.e., effector cells) obtained in Example 1 on B-cell acute lymphoblastic leukemia cells was observed.

*In vitro* killing effect of the universal CAR-T on specific tumor cells:
The experimental steps of the present application were as follows:
Step 1: Target Cell Labeling

Target cells (human Burkitt's lymphoma cells Raji, K562, all cells were from ATCC) were labeled using the CELL TRACE^{™} Far Red Cell Proliferation Kit (manufacturer: Gibco; Cat. No.: 1888569).
1.Dilute Cell Trace^{™} Far Red Cell Proliferation with double distilled water to 1mmol solution;
2.Take 1×10⁶ target cells and centrifuge them at 400g for 5 minutes, then discard the supernatant;
3.Add 1 ul of Cell Trace^{™} Far Red Cell Proliferation solution, and incubate at 37°C for 20min in the dark.
4. Add the cells to T cell culture medium and incubate them at 37°C for 5 min.
5. After centrifugation at 400g for 5 minutes, discard the supernatant, and the labeling was completed.

### Step 2: Detection of target cell killing by effector cells

The labeled target cells were resuspended in R1640+10% FBS medium at a density of 2 × 10⁵ cells/mL, and 500 µL was added to a 48-well plate. According to the appropriate effector-target ratio (2.5:1, 1.25:1, 0.6:1), 500 µL of effector cells were added to each well, and at the same time, T cells and healthy human cord blood CAR-T cells (on the second day of T cell culture, the lentivirus packaged with CAR(the structure of the CAR consists of anti-CD 19 scFv, linker, CD8 α hinge, CD8 transmembrane domain, 4-11BB signaling domain and CD3 zeta), its sequence was shown above, the prepared CAR-T was used as control) was added at a ratio of MOI=2-10 were used as control cells, each group included 3 parallel tests, and an independent target cell group was designed to detect its mortality; cultured at 37°C, 5% CO₂ for 12-16 h, centrifuged at 400 g for 5 min, the cell pellet was taken, and resuspended in 150 ul of DPBS (manufacturer: Gibco; Cat. No.: 1924294). After staining with PI (manufacturer: Sigma; Cat. No.: P4170), the target cell death rate was detected by flow cytometry. The results were shown in Figure 6. Compared with other CAR-T cells, the universal CAR-T was basically the same in killing specific target cells, and the effects were better than T cells. At the same time, it had almost no killing function to non-specific target cells.

### Step 3: ELISA assay of cytokine release

The labeled target cells were resuspended with RPMI 1640+10% FBS at a density of 2×10⁵ cells/mL, and 500 µL of which was added to a 48-well plate. 500 µL of effector cells were added to each well with an appropriate effector-target ratio (10:1), at the same time, T cells and healthy human umbilical cord blood CAR-T were used as control cells, each groupincluded3 parallel tests, and an independent target cell group was designed, and incubated in 5% CO2 for 12-16h. 100ul of the culture supernatant was taken from each well, centrifuged at 400g for 5 minutes to remove the precipitation, and the LEGEND MAXTM Human IL-2/IFN- γ (manufacturer: Biolegend; Cat. Nos.: 431807, 430108) kit was used to detect the release of cytokines after taking the supernatant.

The specific experimental steps were as follows:
1, Dilute the standard sample
2, Wash the plate 4 times with 300ul Wash Buffer (1×) per well, add 50ul Assay Buffer to each well
3, Add 50ul sample to each well
4, Incubate for 2h with shaking at 200rpm
5, Wash the plate 4 times with 300ul Wash Buffer (1×), add 100ul of Detection Antibody solution per well, incubate for 1h with shaking at 200rpm
6, Wash the plate 4 times with 300ul Wash Buffer (1×), add 100ul Svidin-HRP A solution per well, incubate for 30min with shaking at 200rpm
7, Wash the plate 5 times with 300ul Wash Buffer (1×), add Solution F and incubate in the dark for 20 min.
8, Add 100ul Stop Solution per well.
9, Read the absorbance values at 450nm and 570nm with a microplate reader.

The results obtained are shown in Figure 7. In this experiment, Raji was used as the target cell, and T cells, CAR-T and DKO CAR-T+mRNA cells were used as effector cells, after co-culturing *in vitro* with the effect to target ratio of 10:1, 5:1, 2.5:1, 1.25:1, 0.625:1, the supernatant of that with the ratio of 1.25:1 was taken to detect IFN-γ. It can be seen that the killing ability of the universal CAR-T on Raji cells was basically the same as that of common CAR-T or slightly better than that of common CAR-T, in particular, the amount of IFN- γ released by TCRneg CAR-T was much higher than that of common CAR-T, and both have lower lethality to K562, so the release of cytokines was relatively less. This shows the specificity of CAR targeting and does not exhibit the phenomenon of on-target off-tumor.

The above descriptions are only preferred examples of the present application, and are not intended to limit the present application in any form. Any person skilled in the art may use the technical contents disclosed above and change or turn them into the equivalent embodiments with equivalent changes. However, any simple changes, equivalent changes and modifications made to the above examples according to the technical essence of the present application without departing from the content of the technical solutions of the present application still belong to the protection scope of the technical solutions of the present application.

### Sequence Listing

SEQ ID No.1: Cas9 mRNA sequence 
gacaagaaguacagcaucggccuggacaucggcaccaacucugugggcugggccgugaucaccgacgaguacaagg
ugcccagcaagaaauucaaggugcugggcaacaccgaccggcacagcaucaagaagaaccugaucggagcccugcuguuc
gacagcggcgaaacagccgaggccacccggcugaagagaaccgccagaagaagauacaccagacggaagaaccggaucugc
uaucugcaagagaucuucagcaacgagauggccaagguggacgacagcuucuuccacagacuggaagaguccuuccuggu
ggaagaggauaagaagcacgagcggcaccccaucuucggcaacaucguggacgagguggccuaccacgagaaguacccca
ccaucuaccaccugagaaagaaacugguggacagcaccgacaaggccgaccugcggcugaucuaucuggcccuggcccaca
ugaucaaguuccggggccacuuccugaucgagggcgaccugaaccccgacaacagcgacguggacaagcuguucauccag
cuggugcagaccuacaaccagcuguucgaggaaaaccccaucaacgccagcggcguggacgccaaggccauccugucugc
cagacugagcaagagcagacggcuggaaaaucugaucgcccagcugcccggcgagaagaagaauggccuguucggcaacc
ugauugcccugagccugggccugacccccaacuucaagagcaacuucgaccuggccgaggaugccaaacugcagcugagc
aaggacaccuacgacgacgaccuggacaaccugcuggcccagaucggcgaccaguacgccgaccuguuucuggccgccaa
gaaccuguccgacgccauccugcugagcgacauccugagagugaacaccgagaucaccaaggcccccugagcgccucuau
gaucaagagauacgacgagcaccaccaggaccugacccugcugaaagcucucgugcggcagcagcugccugagaaguaca
aagagauuuucuucgaccagagcaagaacggcuacgccggcuacauugacggcggagccagccaggaagaguucuacaag
uucaucaagcccauccuggaaaagauggacggcaccgaggaacugcucgugaagcugaacagagaggaccugcugcggaa
gcagcggaccuucgacaacggcagcaucccccaccagauccaccugggagagcugcacgccauucugcggcggcaggaag
auuuuuacccauuccugaaggacaaccgggaaaagaucgagaagauccugaccuuccgcauccccuacuacgugggcccu
cuggccaggggaaacagcagauucgccuggaugaccagaaagagcgaggaaaccaucaccccuggaacuucgaggaagu
gguggacaagggcgcuuccgcccagagcuucaucgagcggaugaccaacuucgauaagaaccugcccaacgagaaggugc
ugcccaagcacagccugcuguacgaguacuucaccguguauaacgagcugaccaaagugaaauacgugaccgagggaaug
agaaagcccgccuuccugagcggcgagcagaaaaaggccaucguggaccugcuguucaagaccaaccggaaagugaccgu
gaagcagcugaaagaggacuacuucaagaaaaucgagugcuucgacuccguggaaaucuccggcguggaagaucgguuca
acgccucccugggcacauaccacgaucugcugaaaauuaucaaggacaaggacuuccuggacaaugaggaaaacgaggaca
uucuggaagauaucgugcugacccugacacuguuugaggacagagagaugaucgaggaacggcugaaaaccuaugcccac
cuguucgacgacaaagugaugaagcagcugaagcggcggagauacaccggcuggggcaggcugagccggaagcugaucaa
cggcauccgggacaagcaguccggcaagacaauccuggauuuccugaaguccgacggcuucgccaacagaaacuucaugc
agcugauccacgacgacagccugaccuuuaaagaggacauccagaaagcccagguguccggccagggcgauagccugcac
gagcacauugccaaucuggccggcagccccgccauuaagaagggcauccugcagacagugaagguggacgagcucgu
 
gaaagugaugggccggcacaagcccgagaacaucgugaucgaaauggccagagagaaccagaccacccagaagggacagaa
gaacagccgcgagagaaugaagcggaucgaagagggcaucaaagagcugggcagccagauccugaaagaacaccccgugg
aaaacacccagcugcagaacgagaagcuguaccuguacuaccugcagaaugggcgggauauguacguggaccaggaacug
gacaucaaccggcuguccgacuacgauguggaccauaucgugccucagagcuuucugaaggacgacuccaucgacaacaa
ggugcugaccagaagcgacaagaaccggggcaagagcgacaacgugcccuccgaagaggucgugaagaagaugaagaacu
acuggcggcagcugcugaacgccaagcugauuacccagagaaaguucgacaaucugaccaaggccgagagaggcggccug
agcgaacuggauaaggccggcuucaucaagagacagcugguggaaacccggcagaucacaaagcacguggcacagauccu
ggacucccggaugaacacuaaguacgacgagaaugacaagcugauccgggaagugaaagugaucacccugaaguccaagc
ugguguccgauuuccggaaggauuuccaguuuuacaaagugcgcgagaucaacaacuaccaccacgcccacgacgccuac
cugaacgccgucgugggaaccgcccugaucaaaaaguacccuaagcuggaaagcgaguucguguacggcgacuacaaggu
guacgacgugcggaagaugaucgccaagagcgagcaggaaaucggcaaggcuaccgccaaguacuucuucuacagcaaca
ucaugaacuuuuucaagaccgagauuacccuggccaacggcgagauccggaagcggccucugaucgagacaaacggcgaa
accggggagaucgugugggauaagggccgggauuuugccaccgugcggaaagugcugagcaugccccaagugaauaucg
ugaaaaagaccgaggugcagacaggcggcuucagcaaagagucuauccugcccaagaggaacagcgauaagcugaucgcc
agaaagaaggacugggacccuaagaaguacggcggcuucgacagccccaccguggccuauucugugcugguggugccaa
aguggaaaagggcaaguccaagaaacugaagagugugaaagagcugcuggggaucaccaucauggaaagaagcagcuucg
agaagaaucccaucgacuuucuggaagccaagggcuacaaagaagugaaaaaggaccugaucaucaagcugccuaaguac
ucccuguucgagcuggaaaacggccggaagagaaugcuggccucugccggcgaacugcagaagggaaacgaacuggcccu
gcccuccaaauaugugaacuuccuguaccuggccagccacuaugagaagcugaagggcucccccgaggauaaugagcaga
aacagcuguuuguggaacagcacaagcacuaccuggacgagaucaucgagcagaucagcgaguucuccaagagagugauc
cuggccgacgcuaaucuggacaaagugcuguccgccuacaacaagcaccgggauaagcccaucagagagcaggccgagaa
uaucauccaccuguuuacccugaccaaucugggagccccugccgccuucaaguacuuugacaccaccaucgaccggaaga
gguacaccagcaccaaagaggugcuggacgccacccugauccaccagagcaucaccggccuguacgagacacggaucgacc
ugucucagcugggaggcgac
TRAC-sg 2 (T2) sequence: SEQ ID NO: 2
   gcugguacacggcaggguca
TRAC-sg 3 (T3) sequence: SEQ ID NO: 3
   cucucagcugguacacggca
TRAC-sg 4 (T4) sequence: SEQ ID NO: 4
   auuuguuugagaaucaaaau
TRAC-sg 6 (T6) sequence: SEQ ID NO: 5
   ucucucagcugguacacggc
B2M-sg 1(B1) sequence: SEQ ID NO: 6
   acucucucuuucuggccugg
B2M-sg 2(B2) sequence: SEQ ID NO: 7
   gaguagcgcgagcacagcua
B2M-sg 3(B3) sequence: SEQ ID NO: 8
   cgcgagcacagcuaaggcca
B2M-sg 4(B4) sequence: SEQ ID NO: 9
   ucacgucauccagcagagaa
B2M-sg 5(B5) sequence: SEQ ID NO: 10
   gcuacucucucuuucuggcc
B2M-sg 6(B6) sequence: SEQ ID NO: 11
   uuugacuuuccauucucugc
B2M-sg 7(B7) sequence: SEQ ID NO: 12
   cgugaguaaaccugaaucuu
B2M-sg8(B8) sequence: SEQ ID NO: 13
   cucgcgcuacucucucuuuc
Amino acid sequence of anti-TCRα/β scFv: SEQ ID NO: 14 
QIVLTQSPAIMSASPGEKVTMTCSATSSVSYMHWYQQKSGTSPKRWIYDTSKLAS
GVPARFSGSGSGTSYSLTISSMEAEDAATYYCQQWSSNPLTFGAGTKLELKGGGGSGG
GGSGGGGSGGGGSEVQLQQSGPELVKPGASVKMSCKASGYKFTSYVMHWVKQKPG
QGLEWIGYINPYNDVTKYNEKFKGKATLTSDKSSSTAYMELSSLTSEDSAVHYCARGS
YYDYDGFVYWGQGTLVTVSA
Among them, the underlined part is the linker sequence.
Nucleotide sequence of CD8 α hinge region: SEQ ID NO: 15 
accacgacgccagcgccgcgaccaccaacaccggcgcccaccatcgcgtcgcagcccctgtccctgcgcccagaggcgtg
ccggccagcggcgggggcgcagtgcacacgagggggctggacttcgcctgtgat
Nucleotide sequence of CD8 transmembrane region: SEQ ID NO: 16
   atctacatctgggcgcccttggccgggacttgtggggtccttctcctgtcactggttatcaccctttactgc
Nucleotide sequence of the 4-11BB signaling region: SEQ ID NO: 17 
aaacggggcagaaagaaactcctgtatatattcaaacaaccatttatgagaccagtacaaactactcaagaggaagatggctgt
agctgccgatttccagaagaagaagaaggaggatgtgaactg
Nucleotide sequence of CD3 zeta: SEQ ID NO: 18 
agagtgaagttcagcaggagcgcagacgcccccgcgtacaagcagggccagaaccagctctataacgagctcaatctagga
cgaagagaggagtacgatgttttggacaagagacgtggccgggaccctgagatggggggaaagccgagaaggaagaaccctcag
gaaggcctgtacaatgaactgcagaaagataagatggcggaggcctacagtgagattgggatgaaaggcgagcgccggaggggca
aggggcacgatggcctttaccagggtctcagtacagccaccaaggacacctacgacgcccttcacatgcaggccctgccccctcgc
Amino acid sequence of light chain variable region of anti-TCRα/β scFv: SEQ ID NO: 19 
QIVLTQSPAIMSASPGEKVTMTCSATSSVSYMHWYQQKSGTSPKRWIYDTSKLAS
GVPARFSGSGSGTSYSLTISSMEAEDAATYYCQQWSSNPLTFGAGTKLELK
Amino acid sequence of heavy chain variable region of anti-TCRα/β scFv: SEQ ID NO: 20 
EVQLQQSGPELVKPGASVKMSCKASGYKFTSYVMHWVKQKPGQGLEWIGYINP
YNDVTKYNEKFKGKATLTSDKSSSTAYMELSSLTSEDSAVHYCARGSYYDYDGFVY
WGQGTLVTVSA
Amino acid sequence of the linker connecting the light chain variable region and the heavy chain variable region of the scFv: SEQ ID NO: 21
   GGGGSGGGGSGGGGSGGGGS
DNA encoding CD19 light chain: SEQ ID NO: 22 
Gacatccagatgacacagactacatcctccctgtctgcctctctgggagacagagtcaccatcagttgcagggcaagtcagga
cattagtaaatatttaaattggtatcagcagaaaccagatggaactgttaaactcctgatctaccatacatcaagattacactcaggagtcc
catcaaggttcagtggcagtgggtctggaacagattattctctcaccattagcaacctggagcaagaagatattgccacttacttttgcca
acagggtaatacgcttccgtacacgttcggaggggggaccaagctggagatcaca
DNA encoding the linker region: SEQ ID NO: 23
   ggtggcggtggctcgggcggtggtgggtcgggtggcggcggatct
DNA encoding the heavy chain of CD19: SEQ ID NO: 24

Gaggtgaaactgcaggagtcaggacctggcctggtggcgccctcacagagcctgtccgtcacatgcactgtctcaggggtct
cattacccgactatggtgtaagctggattcgccagcctccacgaaagggtctggagtggctgggagtaatatggggtagtgaaaccac
atactataattcagctctcaaatccagactgaccatcatcaaggacaactccaagagccaagttttcttaaaaatgaacagtctgcaaact
gatgacacagccatttactactgtgccaaacattattactacggtggtagctatgctatggactactggggccaaggaacctcagtcacc
gtctcctca

## Claims

1. A method for purifying universal human CAR-T cells, comprising:
(i) disrupting a TRAC genomic region and a B2M genomic region in a human CAR-T cell by gene editing technology; and
(ii) introducing an mRNA targeting TCRα/β into the gene-edited CAR-T cell of step (i).

2. The method according to claim 1, wherein the mRNA targeting TCRα/β is introduced into the CAR-T cell by transient transfection.

3. The method according to claim 1 or 2, further comprising culturing a cell population obtained by the method *in vitro* for an appropriate time to eliminate TCRα/β positive cells.

4. The method according to claim 3, wherein the appropriate time is from 4 to 8 days, preferably from 5 to 7 days, most preferably 6 days.

5. The method according to any one of claims 1 to 4, wherein the TRAC genomic region comprises the genomic region of human chromosome 14 from position 23016448 to 23016490, and the B2M genomic region comprises the genomic region of human chromosome 15 from position 45003745 to 45003788.

6. The method according to any one of claims 1 to 5, wherein the mRNA targeting TCRα/β encodes an anti-TCRα/β scFv molecule, wherein,
a light chain variable region of the anti-TCRα/β scFv molecule is shown in
SEQ ID NO: 19 
(QIVLTQSPAIMSASPGEKVTMTCSATSSVSYMHWYQQKSGTSPKRWIYDTSKLASGVPARFS
GSGSGTSYSLTISSMEAEDAATYYCQQWSSNPLTFGAGTKLELK);
 and
a heavy chain variable region of the anti-TCRα/β scFv molecule is shown in
SEQ ID NO: 20

(EVQLQQSGPELVKPGASVKMSCKASGYKFTSYVMHWVKQKPGQGLEWIGYINPYNDVTK
YNEKFKGKATLTSDKSSSTAYMELSSLTSEDSAVHYCARGSYYDYDGFVYWGQGTLVTVSA)

7. The method according to claim 6, wherein the light chain variable region of the scFv molecule and the heavy chain variable region of the scFv molecule are connected by a linker, and the linker is an amino acid sequence comprising (G)n(S)m, wherein n is a positive integer from 1to 20, and m is a positive integer from 1 to 10.

8. The method according to claim 7, wherein the sequence of the linker is shown in SEQ ID NO: 21: GGGGSGGGGSGGGGSGGGGS.

9. The method according to any one of claims 1 to 8, wherein the amino acid sequence encoded by the mRNA targeting TCRα/β is shown in SEQ ID NO: 14:

QIVLTQSPAIMSASPGEKVTMTCSATSSVSYMHWYQQKSGTSPKRWIYDTSKLASGVPA
RFSGSGSGTSYSLTISSMEAEDAATYYCQQWSSNPLTFGAGTKLELKGGGGSGGGGSGGGGS
GGGGSEVQLQQSGPELVKPGASVKMSCKASGYKFTSYVMHWVKQKPGQGLEWIGYINPYN
DVTKYNEKFKGKATLTSDKSSSTAYMELSSLTSEDSAVHYCARGSYYDYDGFVYWGQGTLV
TVSA.

10. The method according to any one of claims 1 to 9, wherein the TRAC genomic region and the B2M genomic region are disrupted by homologous recombination, zinc finger nucleases gene editing technology, TALEN gene editing technology or CRISPR/Cas gene editing technology.

11. The method according to claim 10, wherein the gene editing technology is CRISPR/Cas9 gene editing technology.

12. The method according to claim 11, comprising
(i) introducing a guide RNA (sgRNA) targeting the TRAC genomic region into a T cell to edit the TRAC genomic region; and/or
(ii) introducing a guide RNA (sgRNA) targeting the B2M genomic region into a T cell to edit the B2M genomic region.

13. The method according to claim 12, wherein:
(i) the guide RNA (sgRNA) targeting the TRAC genomic region has any sequence selected from: SEQ ID NOs: 2 to 5; and
(ii) the guide RNA (sgRNA) targeting the B2M genomic region has any sequence selected from: SEQ ID NOs: 6 to 13.

14. The method according to claim 13, comprising
(i) introducing the sgRNA encoding the TRAC genomic region and a Cas9-encoding nucleotide concurrently or separately into a T cell to edit the TRAC genomic region; and/or
(ii) introducing the sgRNA encoding the B2M genomic region and a Cas9-encoding nucleotide concurrently or separately into a T cell to edit the B2M genomic region.

15. The method according to any one of claims 12-14, wherein the sgRNA is chemically modified.

16. The method according to claim 15, wherein the chemical modification comprises a 2'-O-methyl modification or an internucleotide 3' thiol modification.

17. The method according to claim 16, wherein the chemical modification is a 2'-O-methyl modification at the first one, two and/or three bases at the 5' end and/or the last base at the 3' end of the sgRNA.

18. The method according to any one of claims 14 to 17, wherein the sgRNA and the Cas9-encoding nucleotide sequence are introduced into the T cell together by electroporation.

19. The method according to claim 18, wherein a condition of the electroporation is selected from any one of the following conditions: 150-250V, 0.5-2ms; 150V, 2ms; 160V, 2ms; 170V, 2ms; 180V, 2ms; 190V, 1ms; 200V, 1ms; 210V, 1ms; 220V, 1ms; 230V, 1ms; 240V, 1ms; or 250V, 0.5ms.

20. The method according to any one of claims 1 to 19, wherein the TRAC and B2M are independently knocked out with an efficiency of more than 90%; and collectively knocked out with an efficiency of more than 75%.

21. The method according to any one of claims 1 to 20, wherein the ratio of TCR-negative cells accounts for 98% or more than 99% of all CAR-T cells in the cell population obtained by the method.

22. A purified universal human CAR-T cell, wherein:
(i) the TRAC genomic region and the B2M genomic region in the CAR-T cell have been disrupted by gene editing; and
(ii) an mRNA targeting TCRα/β has been introduced into the CAR-T cell.

23. The universal human CAR-T cell according to claim 22, which expresses at a low level or does not express one or more proteins selected from the group consisting of: TCRα/β, HLA class 1 protein, PD-1, TIM3, and LAG3.

24. A biological product comprising the universal human CAR-T cell of any one of claims 22 or 23.

25. The biological product according to claim 24, wherein 98% or more than 99% of the universal human CAR-T cells are TCR negative.

26. Use of the universal human CAR-T cell according to any one of claims 22 or 23 or the biological product according to any one of claims 24 or 25 in the preparation of a medicament for treating a disease in a subject.

27. A method for treating a disease in a subject, comprising administering to the subject an effective amount of the universal human CAR-T cell according to claim 22 or 23 or the biological product according to claim 24 or 25.

28. The method according to claim 27, wherein the disease is tumor.

29. The method according to claim 28, wherein the tumor is hematological tumor.

30. The method according to claim 29, wherein the tumor is lymphoma or leukemia.
